# EUROPEAN PATENT APPLICATION

(11) **EP 4 832 000 A1**
(43) Date of publication of application: **28.10.2026**
(21) Application number: 25904941.9
(22) Date of filing: 28.11.2025
(51) Int. Cl.: G16H 20/40, G16H 10/60, G16H 40/20, G16H 70/40

(54) **PLATFORM SYSTEM AND METHOD FOR INTEGRATED MANAGEMENT OF INSULIN IN BODY, AND COMPUTER PROGRAM FOR SAME**

(30) Priority: 09.12.2024 KR 20240181927
(71) Applicant: G2E Co., Ltd, Seoul 08394 (KR)
(72) Inventor: JUNG, Chang Bum, Incheon 22714 (KR); LEE, Taemin, Seoul 07297 (KR); YANG, Chung Il, Incheon 22535 (KR); SEO, Changwon, Hanam-si Gyeonggi-do 13008 (KR)
(74) Representative: HGF
(86) International application number: PCT/KR2025/020048
(87) International publication number: WO 2026/127466

(57) **Abstract**

A platform system for integrated management of insulin in body may include: a receiving module configured to receive, through a user input or through a communication connection with one or more injection devices used to administer insulin to a user, insulin administration information including at least one of a past insulin dose and administration timing of the user, and to receive administration means information to be used for future insulin administration of the user; a management module configured to calculate the insulin in body of the user based on the insulin administration information, and, based on the insulin in body and the administration means information, to generate management information including at least one of an insulin dose and administration timing for continuous management of the insulin in body; and an output module configured to provide the management information to the user.

## Description

### [Technical Field]

This patent was filed with the support of the Korea Medical Device Development Fund grant funded by the Korean government (the Ministry of Science and ICT, the Ministry of Trade, Industry and Energy, the Ministry of Health and Welfare, and the Ministry of Food and Drug Safety) (research project title: Advanced development of a pen-type automated insulin delivery (AID) device based on CGM (continuous glucose monitoring)/motion sensor data analysis algorithms; project identification number: 1711196799).

The embodiments relate to a platform system and method for integrated management of insulin in body, and to a computer program for the same. More specifically, the embodiments relate to a technique for providing a user with an optimal insulin management method by calculating and predicting the insulin in body based on the user's insulin administration information, and, based on the predicted insulin in body, proposing a future insulin administration plan to the user or setting it in an injection device.

### [Background Art]

With advances in modern medical technology, diabetic patients have become able to manage blood glucose through insulin administration in various manners. Insulin administration is mainly classified into MDI (Multiple Daily Injection) therapy and CSII (Continuous Subcutaneous Insulin Infusion) therapy. MDI therapy is a method of administering insulin several times a day using an insulin pen or the like, and is preferred by many patients owing to its low initial cost and ease of use. However, when blood glucose management does not reach a target level, it is common to switch from MDI therapy to CSII therapy. CSII therapy is generally a method of continuously administering insulin subcutaneously using an insulin pump, providing the insulin required at each point in time based on a real-time blood glucose value obtained through a continuous glucose monitor, thereby enabling more precise blood glucose management.

Recently, systems that calculate an optimal insulin administration timing and dose based on information such as a patient's blood glucose, diet, and exercise have been introduced. For example, Korean Patent Registration No. 10-2494011 discloses a system that calculates bolus insulin based on a continuous glucose monitor (Continuous Glucose Monitoring; CGM). Bolus insulin refers to insulin that a diabetic patient additionally administers at mealtimes or when blood glucose rises sharply in order to regulate blood glucose; it is mainly a rapid-acting insulin preparation and is used to prevent a sharp rise in blood glucose and to maintain blood glucose within a target range.

However, even a patient who has been managing blood glucose by CSII therapy may, in certain situations, find it difficult to use the insulin pump. For example, in cases such as engaging in vigorous exercise such as marathon running, cycling, or swimming, situations requiring exposure to water such as bathing or sea bathing, or being on a trip or in the presence of others, a situation may arise in which the insulin pump must be removed from the body and an insulin pen must be used for a certain period of time. Conversely, a patient who has been managing blood glucose by MDI therapy using an insulin pen may switch to CSII therapy using an insulin pump in order to improve the level of blood glucose management. In such cases, accurate blood glucose management may become difficult during a transition period of several hours to several days; however, the prior art, including that disclosed in Korean Patent Registration No. 10-2494011, does not provide an integrated management function when the insulin administration characteristics change in this way.

Due to such limitations of the prior art, if the user does not accurately recognize the administration device used for insulin administration and the insulin dose, problems may arise in insulin administration and blood glucose management; and since the user must entirely determine on their own the insulin dose before and after the device is switched, there is a high likelihood of failing to manage blood glucose due to inappropriate judgment. In addition, even when an insulin dose management system utilizing information and communication technology is used, if the history of using mutually different insulin administration means is not managed in an integrated manner, the insulin dose and administration timing may be incorrectly guided based on erroneous information. This is problematic in that it lowers the level of precise blood glucose management, reduces the reliability of the entity operating the system, and can pose a great risk to the health of diabetic patients.

### [Disclosure of the Invention]

### [Technical Problem]

According to one aspect of the present invention for solving such problems of the prior art, there may be provided a platform system for integrated management of insulin in body, a method for integrated management of insulin in body, and a computer program for the same, which have compatibility with various insulin injection devices and which, even when the user uses various administration means such as a pump, an insulin pen, and a medicament, and even when the user changes the administration means being used, calculate and predict the insulin in body (Insulin On Board; insulin in body) based on prior insulin administration information, and thereby enable unified management of insulin administration information.

### [Technical Solution]

A platform system for integrated management of insulin in body according to one aspect of the present invention comprises: a receiving module configured to receive, through a user input or through a communication connection with one or more injection devices used to administer insulin to a user, insulin administration information including at least one of a past insulin dose and administration timing of the user, and to receive administration means information to be used for future insulin administration of the user; a management module configured to calculate the insulin in body of the user based on the insulin administration information, and, based on the insulin in body and the administration means information, to generate management information including at least one of an insulin dose and administration timing for continuous management of the insulin in body; and an output module configured to provide the management information to the user.

In one embodiment, the receiving module is further configured to receive the insulin administration information from a first injection device communicatively connected to the platform system for integrated management of insulin in body. In this case, the management module is further configured to automatically set, based on the management information, at least one of an insulin dose and administration timing of a second injection device communicatively connected to the platform system for integrated management of insulin in body.

In one embodiment, the first injection device and the second injection device are devices having mutually different insulin administration characteristics, and the insulin administration characteristics include at least one of a type of administered insulin, an insulin dose, and an insulin administration cycle. In this case, the management module is further configured to calculate the insulin in body based on the insulin administration information by the first injection device, and, based on the insulin in body and the insulin administration characteristics of the second injection device, to determine at least one of an initial dose and an initial administration time point of insulin by the second injection device.

In one embodiment, the administration means information includes information defining the injection device or medicament to be used by the user for insulin administration. In this case, the management module is further configured to calculate the insulin in body and the management information with respect to bolus insulin or basal insulin administered by the user.

In one embodiment, the management module is further configured to calculate the insulin in body and the management information with respect to each of bolus insulin and basal insulin administered by the user.

A method for integrated management of insulin in body according to one aspect of the present invention comprises: a step in which a platform system for integrated management of insulin in body receives, through a user input or through a communication connection with one or more injection devices used to administer insulin to a user, insulin administration information including at least one of a past insulin dose and administration timing of the user; a step in which the platform system for integrated management of insulin in body calculates the insulin in body of the user based on the insulin administration information; a step in which the platform system for integrated management of insulin in body receives administration means information to be used for future insulin administration of the user; a step in which the platform system for integrated management of insulin in body generates, based on the insulin in body and the administration means information, management information including at least one of an insulin dose and administration timing for continuous management of the insulin in body; and a step in which the platform system for integrated management of insulin in body provides the management information to the user.

In one embodiment, the step of receiving the insulin administration information includes a step in which the platform system for integrated management of insulin in body receives the insulin administration information from a first injection device communicatively connected to the platform system for integrated management of insulin in body. In addition, the step of providing the management information includes a step in which the platform system for integrated management of insulin in body automatically sets, based on the management information, at least one of an insulin dose and administration timing of a second injection device communicatively connected to the platform system for integrated management of insulin in body.

In one embodiment, the step of generating the management information includes a step in which the platform system for integrated management of insulin in body determines, based on the insulin in body calculated based on the insulin administration information by the first injection device and on the insulin administration characteristics of the second injection device, at least one of an initial dose and an initial administration time point of insulin by the second injection device.

In one embodiment, the first injection device is an insulin pump, and the second injection device is an insulin pen.

In this case, the step of determining at least one of the initial dose and the initial administration time point of insulin by the second injection device may include: a step in which the platform system for integrated management of insulin in body calculates, based on the insulin administration information by the first injection device, a current residual insulin in body at the time point of switching to the second injection device; and a step in which the platform system for integrated management of insulin in body determines the initial dose of insulin by the second injection device by reflecting the current residual insulin in body.

In addition, the step of generating the management information may include: a step in which the platform system for integrated management of insulin in body receives information about the time point of switching from the first injection device to the second injection device; and a step in which the platform system for integrated management of insulin in body generates the management information so as to suspend insulin administration by the first injection device during a preset time interval before the switching time point.

In one embodiment, the first injection device is an insulin pen, and the second injection device is an insulin pump.

In this case, the step of determining at least one of the initial dose and the initial administration time point of insulin by the second injection device may include: a step in which the platform system for integrated management of insulin in body calculates a current residual insulin in body based on the insulin administration information by the first injection device; and a step in which the platform system for integrated management of insulin in body delays the administration time point of insulin by the second injection device by reflecting the current residual insulin in body.

In addition, the step of determining at least one of the initial dose and the initial administration time point of insulin by the second injection device may include: a step in which the platform system for integrated management of insulin in body calculates a current active insulin in the body based on the insulin administration information by the first injection device; and a step in which the platform system for integrated management of insulin in body determines the dose of insulin by the second injection device based on an amount obtained by subtracting the current active insulin in the body from a preset target basal active insulin.

In one embodiment, the target basal active insulin is a value at which a sum of active insulin corresponding to long-acting insulin administered a plurality of times at preset time intervals by the first injection device converges.

In one embodiment, the step of determining the dose of insulin by the second injection device includes a step in which the platform system for integrated management of insulin in body linearly increases the insulin dose by the second injection device until an active insulin in the body based on insulin administered into the body by the second injection device reaches the target basal active insulin.

In addition, the insulin administration information by the first injection device may include an administration time point of long-acting insulin by the first injection device. In this case, the step of determining at least one of the initial dose and the initial administration time point of insulin by the second injection device may include a step in which the platform system for integrated management of insulin in body determines the administration time point of insulin by the second injection device based on a duration of action of the long-acting insulin.

In one embodiment, the step of receiving the insulin administration information includes a step in which the platform system for integrated management of insulin in body receives the insulin administration information from an injection device communicatively connected to the platform system for integrated management of insulin in body. In this case, the step of providing the management information further includes: a step in which the platform system for integrated management of insulin in body calculates a current residual insulin in body (IOB) of the user based on the insulin administration information; and a step in which the platform for integrated management of insulin in body determines, based on the current residual insulin in body (IOB), at least one of a dose and an administration time point of a medicament that the user administers for blood glucose regulation.

In one embodiment, the insulin in body and the management information are calculated with respect to bolus insulin or basal insulin administered by the user. In addition, in one embodiment, the insulin in body and the management information may be calculated for each of bolus insulin and basal insulin.

According to still another aspect of the present invention, there is provided a method for integrated management of insulin in body that is performed by a computing device communicating with one or more injection devices so as to provide a continuous management function even when the injection device is switched.

A method for integrated management of insulin in body according to one embodiment comprises: a step of receiving, through a user input or through a communication connection with a first injection device used to administer insulin to a user, first insulin administration information including a past insulin type, dose, and administration timing of the user; a step of calculating, based on the first insulin administration information, a residual insulin in body of the user when the past insulin is rapid-acting, and calculating an active insulin in the body of the user when the past insulin is long-acting; a step of receiving an insulin type corresponding to a second injection device to be used for future insulin administration of the user; and a step of automatically setting, in the second injection device, at least one of an insulin dose and administration timing by the second injection device, based on the calculated residual insulin in body or active insulin in the body of the user and the insulin type corresponding to the second injection device.

In one embodiment, the first injection device and the second injection device are devices having mutually different insulin types.

In this case, the method for integrated management of insulin in body further comprises: a step of receiving, through a user input or through a communication connection with the second injection device, second insulin administration information including an insulin type, dose, and administration timing corresponding to the second injection device; a step of calculating a residual insulin in body or active insulin in the body of the user based on the second insulin administration information; a step of receiving an insulin type corresponding to a third injection device, which is to be used for future insulin administration of the user and which differs in insulin type from the second injection device; and a step of automatically setting, in the third injection device, at least one of an insulin dose and administration timing by the third injection device, based on the residual insulin in body or active insulin in the body of the user calculated based on the second insulin administration information and the insulin type corresponding to the third injection device.

In one embodiment, the first injection device is an insulin pump using rapid-acting insulin, the second injection device is an insulin pen using long-acting insulin, and the third injection device is an insulin pump using rapid-acting insulin.

In this case, the step of automatically setting in the second injection device includes a step of determining at least one of the insulin dose and administration timing of the second injection device, based on a residual insulin in body that is based on the rapid-acting insulin administered into the body by the first injection device and on the first insulin administration information.

In addition, the step of automatically setting in the third injection device includes a step of determining at least one of the insulin dose and administration timing of the third injection device, based on an active insulin in the body that is based on the long-acting insulin administered into the body by the second injection device and on the second insulin administration information.

In still another embodiment, the step of automatically setting in the second injection device includes a step of determining at least one of the insulin dose and administration timing of the second injection device based on a preset target basal active insulin. In this case, the target basal active insulin may be a value at which a sum of active insulin corresponding to long-acting insulin administered a plurality of times at preset time intervals by the first injection device converges.

In addition, in one embodiment, the step of determining at least one of the insulin dose and administration timing of the second injection device includes a step of determining the insulin dose of the second injection device based on a value obtained by subtracting, from the target basal active insulin, a current active insulin in the body that is based on the long-acting insulin administered into the body by the first injection device.

In still another embodiment, the step of determining at least one of the insulin dose and administration timing of the second injection device includes a step of linearly increasing the insulin dose by the second injection device until an active insulin in the body based on the rapid-acting insulin administered into the body by the second injection device reaches the target basal active insulin.

The method for integrated management of insulin in body according to one aspect of the present invention may be stored, in combination with hardware, in a computer-readable recording medium so as to execute the method for integrated management of insulin in body according to the above-described embodiments.

A computer program according to one aspect of the present invention is stored in a computer-readable recording medium so as to execute the method for integrated management of insulin in body according to the above-described embodiments.

### [Advantageous Effects of the Invention]

According to the platform system for integrated management of insulin in body (Insulin On Board; insulin in body) and the method for integrated management of insulin in body according to one aspect of the present invention, a diabetic patient can easily manage his or her insulin administration records through an insulin injection device linked to his or her smartphone app and through data manually entered into the app, and has the advantage of being able to effectively regulate blood glucose by administering insulin in accordance with an optimal insulin administration plan that the system provides based on the insulin in body.

For example, when a patient who has been managing blood glucose by MDI therapy switches to CSII therapy, even if the patient wears the insulin pump immediately after administering insulin via the insulin pen, the platform performs control such as delaying the insulin administration by the pump or adjusting the dose based on the prior insulin administration information, so that the user does not need to attend to each detail in order to switch the administration method. In addition, even when switching from CSII therapy to MDI therapy, the platform automatically calculates and presents the timing and dose at which insulin administration is required, so that the user has the advantage of being able to administer the required amount at an appropriate time in accordance with the guidance of the app.

### [Brief Description of the Drawings]

FIG. 1 is a schematic block diagram of a platform system for integrated management of insulin in body according to one embodiment.
FIG. 2 is a block diagram showing a hardware configuration of a platform system for integrated management of insulin in body according to one embodiment.
FIG. 3 is a schematic block diagram showing a detailed configuration of a receiving module of a platform system for integrated management of insulin in body according to one embodiment.
FIG. 4 is a schematic block diagram showing a detailed configuration of a management module of a platform system for integrated management of insulin in body according to one embodiment.
FIG. 5 is a graph showing a decrease in active insulin and an amount of active insulin over time after insulin administration.
FIG. 6a is a flowchart showing the steps of a method for integrated management of insulin in body according to one embodiment.
FIG. 6b is a flowchart showing the steps of a method for integrated management of insulin in body according to still another embodiment.
FIGS. 7 to 9 are conceptual diagrams showing an exemplary user interface (User Interface; UI) provided by a platform system for integrated management of insulin in body according to one embodiment.
FIGS. 10a to 10d are conceptual diagrams for explaining adjustment of an insulin administration time point by a platform system for integrated management of insulin in body according to one embodiment.

### [Mode for Carrying Out the Invention]

Hereinafter, embodiments of the present invention will be examined in detail with reference to the drawings.

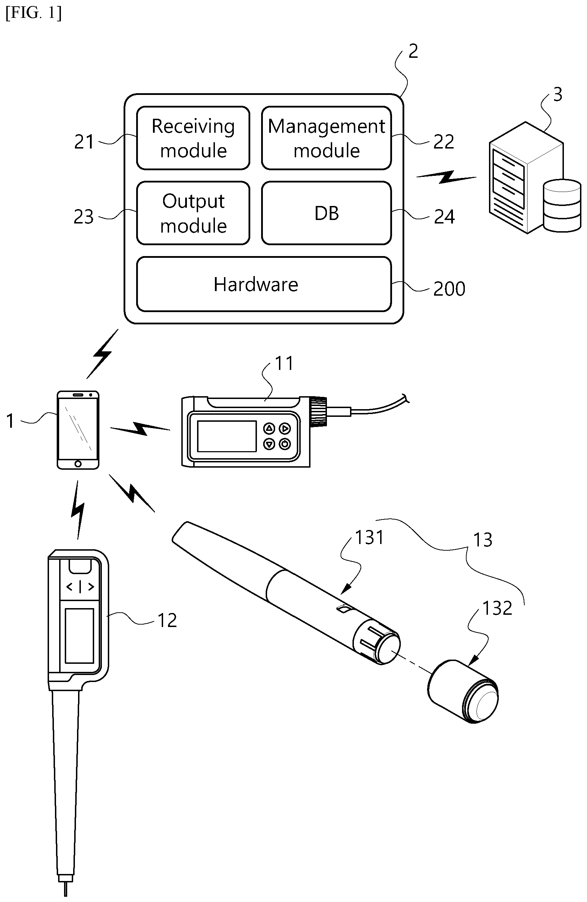

FIG. 1 is a schematic block diagram of a platform system for integrated management of insulin in body according to one embodiment.

Referring to FIG. 1, a platform system for integrated management of insulin in body 2 according to one embodiment may operate while communicating with injection devices 11 to 13 used by a user who needs to administer insulin into his or her own body or another person's body, such as a diabetic patient or a caregiver, and/or with a user device 1 such as a smartphone used by the user.

The number and form of the user devices 1 shown in FIG. 1 are merely exemplary. For example, although FIG. 1 illustrates a single user device 1, a plurality of examiners may each access the platform system for integrated management of insulin in body 2 through their own user device 1. In addition, although the user device 1 is shown in the form of a smartphone in FIG. 1, in other embodiments the user device 1 may be implemented in the form of any computing device, such as another type of mobile communication terminal, a notebook computer, a personal computer, a PDA (personal digital assistant), a tablet, or a set-top box for IPTV (Internet Protocol Television) or the like.

In one embodiment, the platform system for integrated management of insulin in body 2 may further operate while communicating with one or more healthcare servers 3 corresponding to the user. For example, the healthcare server 3 may be a server of a medical institution at which the user receives medical treatment and prescriptions, and in this embodiment the platform system for integrated management of insulin in body 2 may, by communicating with the healthcare server 3, receive information for the user's blood glucose management from the healthcare server 3 or transmit management information generated by the platform system for integrated management of insulin in body 2 to the healthcare server 3.

A user who wishes to perform blood glucose management using the platform system for integrated management of insulin in body 2 may communicatively connect one or more injection devices 11 to 13 used for insulin administration to the platform system for integrated management of insulin in body 2. For example, the injection devices 11 to 13 may be provided with a communication function via a short-range wireless communication network such as Bluetooth, so as to be communicatively connected (or paired) to a user device 1 such as a smartphone.

The user device 1 may transmit information received from the injection devices 11 to 13 to the platform system for integrated management of insulin in body 2 via a short-range or long-range communication network. In addition, the user device 1 may operate to set an insulin administration time point and/or dose by the injection devices 11 to 13 by communicating with the injection devices 11 to 13 based on the management information received from the platform system for integrated management of insulin in body 2.

However, this is exemplary; in other embodiments the platform system for integrated management of insulin in body 2 may be communicatively connected directly to one or more injection devices 11 to 13 without going through the user device 1, or the platform system for integrated management of insulin in body 2 itself may be configured in the form of a software application executed within a user device 1 such as a smartphone, in which case the user device 1 may be omitted.

The platform system for integrated management of insulin in body 2 according to the embodiments may, by communicating with the user device 1 and/or one or more injection devices 11 to 13 via a wired and/or wireless network, receive insulin administration information defining the user's insulin type, insulin dose, and/or administration time point, calculate the user's insulin in body based on the received information, and provide a user interface (User Interface; UI) for displaying management information that enables the user's insulin in body to be managed at a constant level despite changes in the user's administration environment.

In this specification, administration of insulin is intended, unless otherwise limited, to encompass the act of injecting liquid-form insulin into the body using an injector, or the act of orally administering a medicament for blood glucose regulation.

In this specification, the injection devices 11 to 13 refer to any device that may be used to inject insulin into the user's body and that is provided with an information communication function with the user device 1 and/or the platform system for integrated management of insulin in body 2. In this specification, the injection devices 11 to 13 are described by way of example as an insulin pen 12 and an insulin pen cap 13 to be used in MDI (Multiple Daily Injection) therapy, and an insulin pump 11 to be used in CSII (Continuous Subcutaneous Insulin Infusion) therapy; however, the type and form of the injection devices 11 to 13 that may be used together with the platform system for integrated management of insulin in body 2 according to the embodiments are not limited thereto.

For example, the insulin pen described as the injection devices 11 to 13 in this specification is intended as a concept encompassing not only a general reusable pen but also a disposable pen, a disposable syringe, an insulin pen cap, and the like. In addition, the insulin pen 12 may be of a dial type in which a dose is determined by turning a dial, or may be of a different type. In this case, the insulin pen cap 13 is equipment having a wireless communication function that is coupled to a general manual insulin pen; when a dial is turned on the manual insulin pen 131 to set a dose, a pen cap 132, which is an electronic device detachably coupled to the manual insulin pen 131, automatically recognizes the dial, stores the dose and the administration time point, and may transmit such stored information to the user device 1 and/or the platform system for integrated management of insulin in body 2 via wireless communication. In addition, a general insulin injection therapy may also be included in the injection device of the present invention if the insulin in body is manageable.

Meanwhile, the insulin pump 11 described as the injection devices 11 to 13 in this specification may be equipped with an algorithm that is linked with continuous glucose monitoring to suspend insulin administration upon or before reaching hypoglycemia, or may simply administer insulin at a fixed dose and cycle. In addition, the insulin pump may be configured to administer insulin subcutaneously in various manners, such as a needle type or a patch type, and is not limited to having a particular hardware configuration.

In the embodiments of the present invention, the insulin that the user administers via the injection devices 11 to 13 may be bolus insulin (Bolus Insulin), may be basal insulin (Basal Insulin), or may be a mixed-type insulin in which these functions are combined.

In this specification, bolus insulin is used to regulate a meal-related blood glucose surge, and refers to rapid-acting or ultra-rapid-acting insulin that is usually administered immediately before or immediately after a meal. Throughout this specification, where the expression 'rapid-acting' is used, it refers to both 'rapid-acting insulin' and 'ultra-rapid-acting insulin'; for example, bolus insulin is an insulin that acts quickly, beginning to take effect within about 15 minutes after administration, reaching a peak blood concentration after about 1 hour, and lasting in effect for about 2 to 4 hours, and may be, but is not limited to, insulin aspart, insulin lispro, insulin glulisine, or the like.

In addition, in this specification, basal insulin refers to insulin that is used to maintain a constant blood glucose throughout the day, and that is a type of insulin which is slowly absorbed and acts over a long period of time. Basal insulin is usually administered once or twice a day and ordinarily provides an effect lasting for 24 hours, and may be, but is not limited to, insulin degludec, insulin glargine, insulin detemir, or the like.

The platform system for integrated management of insulin in body 2 according to the embodiments may, for each type of insulin, calculate the insulin in body resulting from prior administration of that insulin, and, based on the calculated insulin in body, manage the timing and/or amount at which the same or a different type of insulin should be administered in the future.

In this specification, the insulin in body denotes an amount of insulin that remains in the user's body and that affects the user's future blood glucose value. In one embodiment, the insulin in body may be defined in mutually different forms depending on the type of insulin that the user has administered in the past.

For example, when the user has used rapid-acting insulin (including ultra-rapid-acting insulin) in the past, the user's insulin in body may be calculated as a residual insulin in body (Insulin on Board; IOB), which denotes an amount of the insulin that has been administered into the body and that still remains. For example, since rapid-acting insulin acts for several hours after administration, the IOB, which is the amount of insulin that has not yet acted and remains, may be utilized as insulin in body information.

Alternatively, when the user has used long-acting insulin in the past, the user's active insulin in the body (active insulin, U/min) may be used as the insulin in body information. Long-acting insulin acts for one to several days and has a relatively constant action profile compared with rapid-acting insulin. In this case, in the embodiments, the amount of active insulin per unit time calculated through the action profile of the long-acting insulin may be calculated as the active insulin in the body and utilized as the insulin in body information.

The platform system for integrated management of insulin in body 2 according to the embodiments may, when the user administers one or more types of insulin, provide a management function independently or in combination for each type of insulin (for example, bolus insulin and basal insulin) and its injection device. In addition, the platform system for integrated management of insulin in body 2 according to the embodiments may provide a management function based on the insulin in body in response to a case where the user uses one or more types of administration means (such as an injection device or oral administration) in parallel or switches the administration means.

For example, by applying the platform system for integrated management of insulin in body 2 according to the embodiments to cases such as where the user switches from an insulin pump using rapid-acting insulin to an insulin pen using long-acting insulin, and where the user switches from an insulin pen using long-acting insulin to an insulin pump using rapid-acting insulin, the dose and/or administration timing of insulin to be administered in the future may be determined based on the type, dose, and/or administration timing of the insulin administered in the past. In addition, the platform system for integrated management of insulin in body 2 may automatically set the administration information determined based on the insulin in body in the injection devices 11 to 13 through direct or indirect communication with the injection devices 11 to 13.

The user may communicatively connect the injection devices 11 to 13 and the user device 1 by pairing via a short-range communication network such as Bluetooth, and may receive insulin administration information via the injection devices 11 to 13 at the user device 1. In addition, the insulin administration information received at the user device 1 may be transmitted from the user device 1 to the platform system for integrated management of insulin in body 2 by the user's selection or automatically.

Meanwhile, when the platform system for integrated management of insulin in body 2 is communicatively connected directly to the injection devices 11 to 13, the platform system for integrated management of insulin in body 2 may receive the insulin administration information from the injection devices 11 to 13. Since accurate insulin administration information by the injection devices 11 to 13 is transmitted to the platform system for integrated management of insulin in body 2, there is an advantage of being able to prevent erroneous entry that may occur when the user manually inputs an insulin dose such as bolus insulin.

However, in the case of another administration means with which communication with the user device 1 is impossible or with which a communication connection has not been established, the user may directly input the insulin administration information via that administration means into the platform system for integrated management of insulin in body 2. For example, the user may input, into the user device 1, the insulin dose and administration time point via another insulin pen or insulin pump that is not paired with the user device 1, so that the input information is transmitted from the user device 1 to the platform system for integrated management of insulin in body 2. In addition, in addition to a device equipped with an insulin injection function, the user may input, into the user device 1, administration information about a blood glucose-lowering agent that the user has administered via a medicament.

For the above operations, the platform system for integrated management of insulin in body 2 may include an application service server that enables operation of a predetermined application (or app) executed on the user device 1, or may include a web server that provides a predetermined web page accessible via a web browser executed on the user device 1. The user may use the service provided by the platform system for integrated management of insulin in body 2 by executing a particular app or accessing a particular web page on his or her smartphone.

However, the depiction of the platform system for integrated management of insulin in body 2 and the user device 1 as separate devices in the drawings attached to this specification is merely exemplary; depending on the embodiment, the platform system for integrated management of insulin in body 2 may be implemented at least partially in the form of a software application stored and executed on the user device 1.

In one embodiment, the platform system for integrated management of insulin in body 2 includes a receiving module 21, a management module 22, and an output module 23. In addition, in one embodiment, the platform system for integrated management of insulin in body 2 may further include a database (DB) 24 for storing information such as blood glucose management records relating to one or more patients using the platform system for integrated management of insulin in body 2. Furthermore, each of these modules 21 to 23 and the DB 24 may be realized at least partially through a combination of the hardware 200 configuration and software of the platform system for integrated management of insulin in body 2.

That is, the devices described in this specification may be entirely hardware, or may have aspects that are partly hardware and partly software. For example, the platform system for integrated management of insulin in body 2 according to the embodiments and each system, device, and server communicating therewith, as well as each unit included therein, may collectively refer to a device for exchanging data of a particular form and content by electronic communication and to software related thereto. In this specification, terms such as "unit," "module," "server," "system," "platform," "device," or "terminal" are intended to refer to a combination of hardware and software driven by that hardware. For example, here, the hardware may be a data processing apparatus including a CPU or another processor. In addition, the software driven by the hardware may refer to a running process, an object, an executable, a thread of execution, a program, or the like.

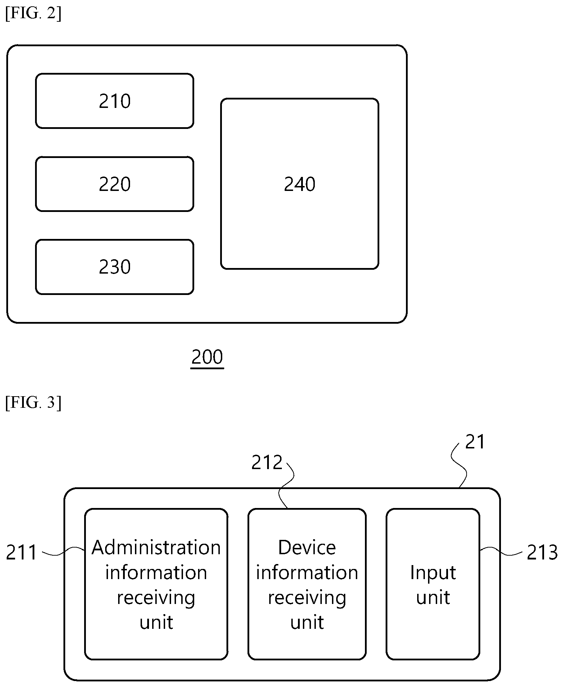

In addition, each element constituting the platform system for integrated management of insulin in body 2 is not necessarily intended to refer to a separate device that is physically distinct from one another. That is, each module and DB of the platform system for integrated management of insulin in body 2 shown in FIG. 2 merely functionally divides the hardware constituting the platform system for integrated management of insulin in body 2 according to the operations performed by that hardware, and the respective components are not necessarily required to be provided independently of one another. Of course, depending on the embodiment, it is also possible for one or more of the aforementioned servers, units, DBs, or modules to be implemented as separate devices that are physically distinct from one another.

The receiving module 21 may receive information such as the type of insulin administered in the past, the past insulin dose, and the administration timing, through information input from the user or through communication with the injection devices 11 to 13 that administer insulin to the user. For example, when the user inputs an insulin administration record into the user device 1 via a smartphone app, this information may be transmitted to the receiving module 21.

In addition, the receiving module 21 may receive administration means information to be used for future insulin administration of the user. The administration means to be used for future insulin administration of the user may be information about an injection device 11 to 13 that has been newly paired with the user device 1 or selected by the user as the means currently in use, or may be information defining an insulin injection device or an orally administered medicament that the user has directly designated. Alternatively, the information that the receiving module 21 receives in relation to future insulin administration may define only the type of insulin that the user will administer in the future.

The management module 22 may calculate the user's insulin in body based on the insulin administration information received through the receiving module 21, and may generate management information designating a future insulin dose and/or administration timing based on the insulin in body according to the previously administered insulin and on the information about the insulin administration means that the user will use in the future. For example, in the case of rapid-acting insulin, the management module 22 may calculate the residual insulin in body (IOB) of the insulin that the user has administered up to the previous day and the time point at which that insulin acts in the body (DIA), and thereby calculate the dose and administration timing of insulin that the user should administer at the present or a near time point.

The output module 23 serves to provide the user with the management information calculated by the management module 22 based on the insulin in body. In one embodiment, the management information may be transmitted to a user device 1 capable of communicating with the platform system for integrated management of insulin in body 2, and may be delivered to the user on the user device 1 in various manners such as a digital display, a notification, or a voice guidance. For example, a smartphone app may deliver, as a notification, the insulin dose that the user needs.

In still another embodiment, the output module 23 may transmit the management information directly to the injection devices 11 to 13 communicatively connected to the platform system for integrated management of insulin in body 2, or the user device 1 that has received the management information from the platform system for integrated management of insulin in body 2 may control the injection devices 11 to 13, so that the next insulin dose and administration timing according to the integratedly managed insulin in body are automatically set in the injection devices 11 to 13.

Through the above operations, the platform system for integrated management of insulin in body 2 may provide a continuous management function based on the insulin in body information even when the device that the user uses for insulin administration is changed.

FIG. 2 is a block diagram showing a hardware configuration of a platform system for integrated management of insulin in body according to one embodiment.

Referring to FIG. 2, the hardware 200 constituting the platform system for integrated management of insulin in body according to the embodiments may be implemented as a computing device including one or more hardware components. In this case, the hardware 200 may include a memory 210, a processor 220, and a communication module 230. In one embodiment, the hardware 200 may further include an input/output unit 240.

The memory 210, as a non-transitory computer-readable recording medium, may include a permanent mass storage device such as a RAM (random access memory), a ROM (read only memory), a disk drive, an SSD (solid state drive), or a flash memory. Here, a permanent mass storage device such as a ROM, an SSD, a flash memory, or a disk drive may be included in the aforementioned device or server as a separate permanent storage device distinct from the memory 210.

In addition, an operating system and at least one program code (for example, code for executing an application installed and run on a server, a user device, or the like) may be stored in the memory 210. Such software components may be loaded from a computer-readable recording medium separate from the memory 210. Such a separate computer-readable recording medium may include a computer-readable recording medium such as a floppy drive, a disk, a tape, a DVD/CD-ROM drive, or a memory card.

In other embodiments, the software components may be loaded into the memory 210 through the communication module 230 rather than from a computer-readable recording medium. For example, at least one program may be loaded into the memory 210 based on a computer program that is installed by files provided over a network by developers or a file distribution system that distributes installation files of applications (for example, a commercial application store service server).

The processor 220 may be configured to process instructions of a computer program by performing basic arithmetic, logic, and input/output operations. The instructions may be provided to the processor 220 by the memory 210 or the communication module 230. For example, the processor 220 may be configured to execute received instructions according to program code stored in a recording device such as the memory 210.

The communication module 230 may provide a function for the platform system for integrated management of insulin in body to communicate with a user device, an insulin injection device, a healthcare server, and the like over a network. That is, the communication module 230 is a part for realizing the operation of each functional module described above with reference to FIG. 2, with its function being controlled by the processor 220 that references the memory 210.

The input/output unit 240 may be a means for interfacing with an external input/output device (not shown). For example, the external input device may include a device such as a keyboard, a mouse, a microphone, or a camera, and the external output device may include a device such as a display, a speaker, or a haptic feedback device. As another example, the input/output unit 240 may be a means for interfacing with a device, such as a touchscreen, in which functions for input and output are integrated into one.

In addition, in other embodiments, the hardware 200 may include more components than those shown in FIG. 2 depending on the nature of the device to which it is applied. For example, when the hardware 200 is applied to a user device, it may be implemented to include at least some of the input/output devices described above, or may further include other components such as a transceiver, a GPS (Global Positioning System) module, a camera, various sensors, and a database. As a more specific example, when the user device is a smartphone, it may be implemented to further include various components generally included in a smartphone, such as an acceleration sensor or a gyro sensor, a camera module, various physical buttons, buttons using a touch panel, an input/output port, and a vibrator for vibration.

FIG. 3 is a schematic block diagram showing a detailed configuration of a receiving module of a platform system for integrated management of insulin in body according to one embodiment.

Referring to FIG. 3, in one embodiment, the receiving module 21 of the platform system for integrated management of insulin in body may include an administration information receiving unit 211, a device information receiving unit 212, and an input unit 213. The device information receiving unit 212 is a part that receives information about an insulin administration means equipped with a communication function, such as an insulin pen or pump used by the user, and may, for example, receive information of an injection device paired with the user device 1 through communication with the user device 1 such as the user's smartphone. In addition, the information of the device that the user uses for insulin injection may be stored and managed in the DB of the platform system for integrated management of insulin in body.

The administration information receiving unit 211 may receive insulin administration information indicating the type, dose, and administration time point of insulin that the user has administered with an injection device in the past. For example, the insulin administration information may be transmitted from the injection device to the paired user device, and then transmitted from the user device 1 to the receiving module 21 of the platform system for integrated management of insulin in body.

Alternatively, the user may manually input, through his or her user device 1, the type of insulin administered, the time point of insulin administration, and/or the dose, and the input unit 213 of the platform system for integrated management of insulin in body may receive the information thus directly input by the user as the insulin administration information.

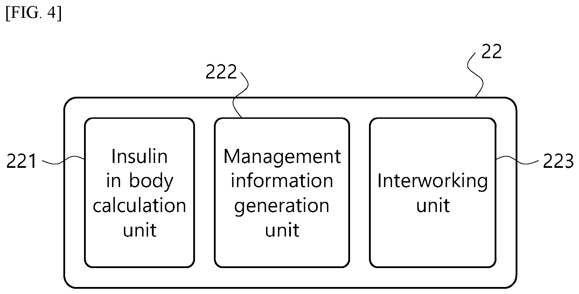

FIG. 4 is a schematic block diagram showing a detailed configuration of a management module of a platform system for integrated management of insulin in body according to one embodiment.

Referring to FIG. 4, in one embodiment, the management module 22 of the platform system for integrated management of insulin in body may include an insulin in body calculation unit 221, a management information generation unit 222, and an interworking unit 223. The insulin in body calculation unit 221 is a part that generates insulin in body information based on the dose and administration timing of insulin that the user has administered in the past. In this case, the insulin in body calculated by the insulin in body calculation unit 221 may differ as a residual insulin in body (IOB) or an active insulin in the body depending on the type of insulin used in the past.

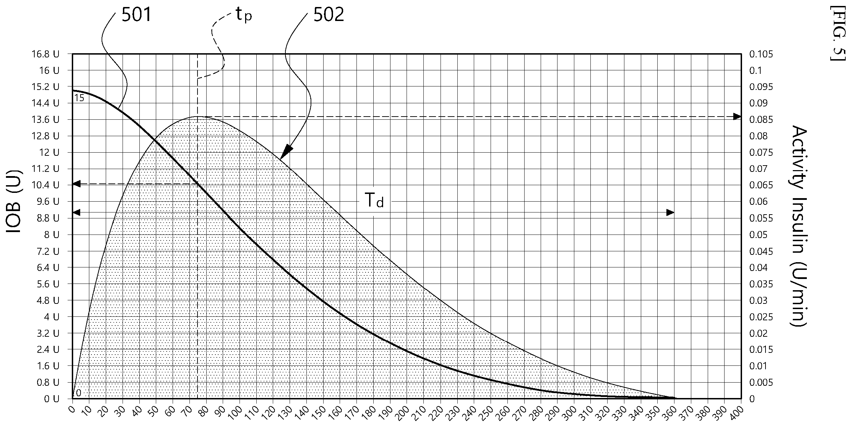

In one embodiment, when the user has administered rapid-acting insulin in the past, the insulin in body calculation unit 221 calculates a current IOB and a target basal active insulin. The current IOB denotes residual insulin that decreases in the body over time after the past administration time point and remains in the body at the present time point. Such a current IOB may be calculated as a decreasing curve starting from the past administration time point using the following items, and FIG. 5 is a graph showing the decrease in residual insulin in body (IOB) and the active insulin (active insulin, U/min) over time after insulin administration. In FIG. 5, a first graph 501 represents the residual insulin remaining in the body, and a second graph 502 represents the active insulin with respect to the body. As shown, the residual insulin in body 501 gradually decreases over the duration of insulin activity (Duration of Insulin Activity; DIA) (Td) after insulin administration, and the active insulin in the body 502 increases after insulin administration, forms a peak at the peak time (Peak Time) (tp), and then gradually decreases.

The current IOB remaining in the body is, when meal bolus insulin is to be administered, subtracted at an appropriate level from the calculated insulin dose so as to prevent hypoglycemia. In this case, the proportion by which the current IOB is subtracted from the insulin dose may be appropriately selected. In one embodiment of the present invention, the IOB Calculations of OpenAPS were used to calculate the current IOB. For example, the current IOB was calculated by reflecting the following variables, but the calculation method is not limited thereto.
Duration of insulin activity (DIA) - ultra-rapid-acting: 240 minutes, rapid-acting: 360 minutes
Peak time (Peak Time) - 75 minutes

In this case, the residual insulin in body 501 is shown as rapid-acting insulin having a duration of insulin activity of 360 minutes, all of which is depleted at 360 minutes along the horizontal time axis after the initial administration. As shown, if 15 U of rapid-acting insulin was initially administered into the body, it can be confirmed that at 75 minutes, when the active insulin in the body 502 is at its maximum, the residual insulin in body 501 is calculated as 10.4 U, indicating that the amount of insulin remaining in the body has decreased.

In addition, the active insulin in the body 502 has a unit of U/min and represents the amount of insulin acting per unit time. The active insulin in the body 502 increases after insulin administration, forms a peak at the peak time (Peak Time) (tp), and then gradually decreases. As shown, if 15 U of rapid-acting insulin was administered into the body, it can be seen that at 75 minutes, when the active insulin in the body 502 is at its maximum, it acts at 0.085 U/min.

Meanwhile, although FIG. 5 calculates and graphically represents the IOB of rapid-acting insulin having a duration of insulin activity of 360 minutes, this calculation method may be equally applied not only to rapid-acting insulin but also to the IOB calculation of long-acting insulin or the active insulin calculation of long-acting insulin.

The target basal active insulin denotes a target value for managing the dose of basal insulin administered by the user through various administration means, and may be a single fixed value, may be a numerical range including an upper limit and/or a lower limit, may be a dose obtained by dividing, by the number of administrations, a range of the total amount of basal insulin that may be determined according to the time of day and the activity state (diet, exercise, stress, sleep, and the like), or may be defined in the form of a combination of different doses so as to match the total amount of basal insulin. In particular, the target basal active insulin may be used when switching from an insulin pen to an insulin pump, and in particular may be used for the purpose of managing the total amount of basal insulin per period.

The management information generation unit 222 may generate, based on the insulin in body calculated by the insulin in body calculation unit 221, management information that indicates the dose and/or administration time point of insulin that the user should administer in the future. If there is received information about the insulin administration means that the user will use in the future (including administration via an injection device and/or administration by a medicament, and including the type of insulin), the management information generation unit 222 may generate the management information based on the administration characteristics of that administration means (for example, the dose per administration, the administration cycle, and the like).

When the administration means to be used in the future is not separately specified, the management information generation unit 222 may generate the management information so as to achieve the target basal active insulin of the user on the premise that the previously used administration means continues to be used.

The interworking unit 223 serves to enable insulin in body and blood glucose management based on the management information to be actually realized, by the management information generated by the management information generation unit 222 being presented to the user or set in the injection device used by the user. For example, the interworking unit 223 may transmit the management information to a user device communicatively connected to the platform system for integrated management of insulin in body. Alternatively, the interworking unit 223 may set administration information directly in the injection device through communication between the platform system for integrated management of insulin in body and the injection device, or may transmit administration information for setting the injection device to the user device so that the user device automatically sets the administration information in the injection device paired therewith.

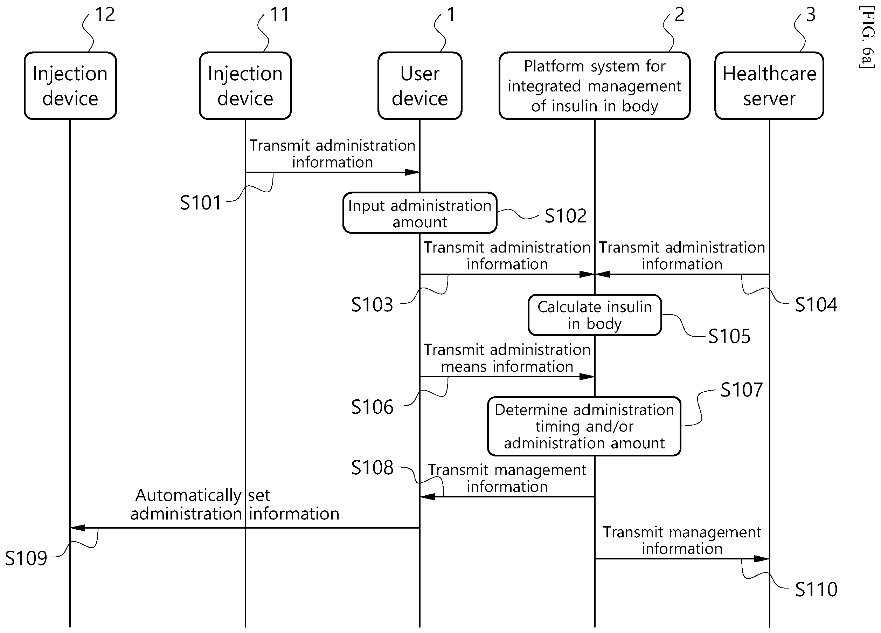

FIG. 6a is a flowchart showing the steps of a method for integrated management of insulin in body according to one embodiment.

Referring to FIG. 6a, when the user uses an injection device 11 to 12 having a communication function over a network, the user may receive insulin administration information by the injection device 11 to 12 at the user device 1 by pairing the user device 1, such as his or her smartphone, with the injection device 11 to 12 (S101). For example, the insulin administration information may include data on the type of insulin, the amount of insulin, and/or the administration timing of the insulin administered into the body by the injection device 11 to 12, such as an insulin pen or an insulin pump.

In one embodiment, when the injection device 11 to 12 is communicatively connected to the user device 1 or the platform system for integrated management of insulin in body 2, the time of the injection device 11 to 12 may be synchronized with the time of the platform system for integrated management of insulin in body 2 for consistent insulin in body management. In addition, the platform system for integrated management of insulin in body 2 may store, in the DB of the platform system for integrated management of insulin in body 2, information of the injection device 11 to 12 registered in relation to the user, such as device identification information, remaining battery level, remaining insulin level, insulin expiration date, insulin administration history, and/or device status information.

In parallel with or in place of the administration information received from the injection device 11 to 12, the user may directly input the type of insulin, the amount of insulin, and/or the administration timing into the user device 1 (S102). For example, the user may input, into the user device 1, the insulin administration timing and dose using another injection device that is not paired with the user device 1, or the type, dose, and administration timing of an administered medicament. In addition, if the user inputs only the insulin dose, or only the type and amount of the medicament, into the user device 1, the administration information may be stored by automatically setting the time point at which that input was received as the administration timing.

The user device 1 may transmit the administration information received from the first injection device 11 that the user used in the past and/or the administration information directly input by the user to the platform system for integrated management of insulin in body 2 (S103). For example, the user may transmit the insulin administration information to the platform system for integrated management of insulin in body 2, which corresponds to an application service server, via an app executed on the smartphone.

However, this is exemplary; in other embodiments the platform system for integrated management of insulin in body 2 may be communicatively connected to the first injection device 11 and receive the insulin administration information directly from the first injection device 11 without going through a user device 1 such as a smartphone.

In addition, in one embodiment, the platform system for integrated management of insulin in body 2 may receive the user's insulin administration information from one or more healthcare servers 3 (S104). For example, the healthcare server 3 may be a device of a medical institution at which the user, who is a diabetic patient, periodically receives medical treatment and prescriptions, and the platform system for integrated management of insulin in body 2 may receive, from the healthcare server 3 as insulin administration information, the medical institution's medical records, or part of the medical records indicating the user's insulin management state or blood glucose management state (S104).

Next, the platform system for integrated management of insulin in body 2 may calculate the user's insulin in body based on the received insulin administration information (S105). This may refer to a process of analyzing the user's past insulin administration data to calculate the insulin in body, which is the amount of active insulin currently remaining in the body. For example, when the insulin used in the past is rapid-acting insulin, the insulin in body may be calculated as a current IOB, or when the insulin used in the past is long-acting insulin, the insulin in body may be calculated as an active insulin in the body.

In addition, at this time, the platform system for integrated management of insulin in body 2 may further calculate or adjust the user's target basal active insulin in addition to the insulin in body. The target basal active insulin denotes a numerical calculation of the basal insulin that the user wishes to maintain, based on the user's past meal pattern, insulin administration pattern, blood glucose fluctuation pattern by time of day, and blood glucose fluctuation pattern by activity situation, and the like, and may be defined in various forms such as a lower limit value, an upper limit value, or an average value of the amount of active insulin, or a numerical range defined by upper and lower limits. Such a target basal active insulin may be calculated using the insulin administration information received from the healthcare server 3.

When a user who has been using the platform system for integrated management of insulin in body 2 wishes to change the means by which the user administers insulin, the platform system for integrated management of insulin in body 2 may receive, from the user device 1, information about the administration means that the user wishes to use for future insulin administration (S106). In this case, the administration means information may include the type of insulin that the user will use in the future.

For example, the administration means may be an injection device such as an insulin pen or a pump, and device information manually input by the user into the user device 1 or device information of an injection device 11 to 12 newly paired with the user device 1 may be transmitted to the platform system for integrated management of insulin in body 2 as the administration means information. Alternatively, when the user administers a blood glucose-improving agent, if the user inputs information about the medicament that the user intends to take into the user device 1, such medicament information may be transmitted to the platform system for integrated management of insulin in body 2 as the administration means information.

By using an app that communicates with the platform system for integrated management of insulin in body 2, the user may directly change the injection device 11 to 12 linked to that app and thereby notify the platform system for integrated management of insulin in body 2 of the switching of the administration means. In addition, when the communication connection between the user device 1 and the previously paired injection device 11 to 12 is disconnected, the app on the user device 1 automatically searches for a pairable injection device 11 to 12 within communication range, and if there is an injection device 11 to 12 newly paired as a result of the search, the information of that injection device 11 to 12 may be transmitted to the platform system for integrated management of insulin in body 2.

In the platform system for integrated management of insulin in body 2, when long-acting insulin is used as basal insulin via an insulin pen, integrated management is performed based on the received past insulin administration information and the information on the administration means that the user wishes to use, so that the target basal active insulin can be achieved regardless of which insulin administration means the user uses. For example, the platform system for integrated management of insulin in body 2 may determine the future insulin administration timing and/or dose based on the insulin administration means to be used in the future and the user's target basal active insulin (S107), and may provide the determined information to the user as management information.

The management information for the integrated management of insulin in body may be transmitted from the platform system for integrated management of insulin in body 2 to the user device 1 (S108). The user may administer insulin himself or herself while complying with the insulin administration timing and/or dose defined in the management information provided from the platform system for integrated management of insulin in body 2, or may set the management information in a second injection device 12 to be used in the future so that the second injection device 12 administers insulin as instructed in the management information.

Alternatively, the management information is transmitted from the platform system for integrated management of insulin in body 2 to the user device 1, and the app executed on the user device 1 may automatically set the administration information of the second injection device 12 paired with the user device 1 based on the received management information, so that the operation of the second injection device 12 is controlled in accordance with the integratedly managed insulin in body even without the user taking any separate action.

In this case, the parameters for the operation of the second injection device 12 may not only set the type, administration time point, and/or dose of insulin to be administered into the user's body via the second injection device 12, but may also be determined by reflecting the user's blood glucose level before the switching of the administration means, the user's insulin sensitivity, the user's past insulin administration history, and a treatment plan received from a medical institution in relation to the user. In this case, the platform system for integrated management of insulin in body 2 stores the aforementioned information relating to the user as variables in the DB of the platform system for integrated management of insulin in body 2, and may generate management information to be transmitted to the injection device 11 to 12 by reflecting those variables.

In one embodiment, the management information transmitted by the platform system for integrated management of insulin in body 2 may, in addition to directly setting the operation of the injection device 11 to 12 to be used by the user, include data instructing the user to perform a corresponding action necessary for continuous management of the insulin in body in order to prepare for switching to use of that injection device 11 to 12. For example, for blood glucose management while the switching of the injection device 11 to 12 takes place, data on the type, administration time point, and/or dose of insulin that the user should administer in advance before the device switching may be generated as management information. In this case, the management information for advance response may be calculated by taking into account the user's current blood glucose value before the device switching, the blood glucose trend, the time required for the device switching, and the like.

In addition, in one embodiment, the platform system for integrated management of insulin in body 2 may further transmit, to the user device 1, notification information that urges the user to take action for continuous management of the insulin in body calculated based on the insulin administration information. For example, when depletion of the insulin in body is expected but the second injection device 12 has not been connected to the user device 1 after the connection between the first injection device 11 and the user device 1 has been disconnected, the platform system for integrated management of insulin in body 2 may generate and transmit to the user device 1 notification information urging the user to pair another injection device with the user device, to administer insulin using a separate insulin injection device, or, if insulin has already been administered, to input the administration information.

In addition, in one embodiment, the platform system for integrated management of insulin in body 2 may further transmit the management information determined based on the integratedly managed insulin in body to a healthcare server 3 such as a medical institution corresponding to the user or the National Health Insurance Service (S110). The healthcare server 3 may, based on the management information received from the platform system for integrated management of insulin in body 2, ascertain the management therapy or insulin dose that the user is currently applying for blood glucose management, and use this for future disease or health management.

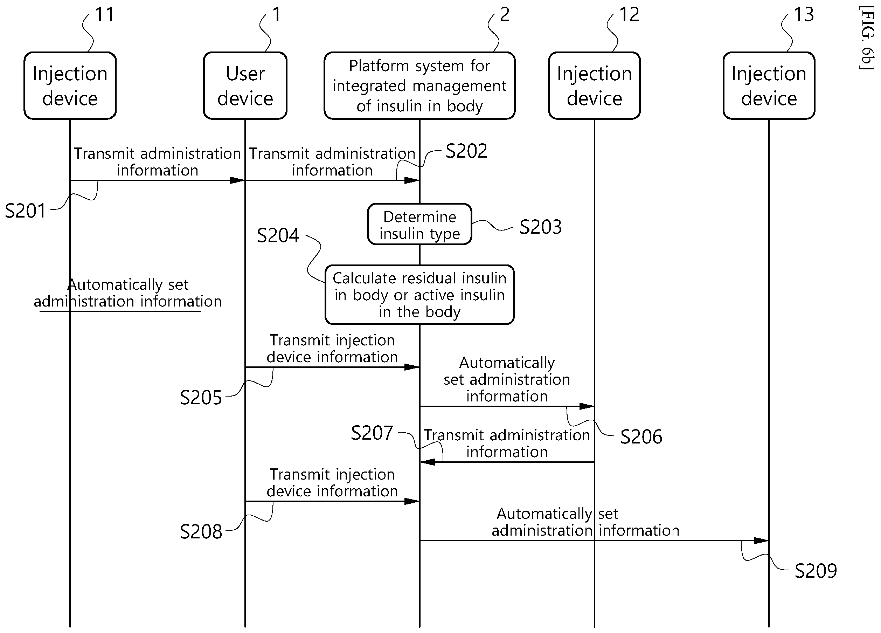

FIG. 6b is a flowchart showing the steps of a method for integrated management of insulin in body according to still another embodiment.

Referring to FIG. 6b, the first injection device 11 transmits administration information defining the past insulin type, dose, and/or administration time point to the paired user device 1 (S201), and the user device 1 may transmit this to the platform system for integrated management of insulin in body 2 (S202). However, as described above, in other embodiments the platform system for integrated management of insulin in body 2 may be communicatively connected to the first injection device 11 and receive such information directly from the first injection device 11.

The platform system for integrated management of insulin in body 2 may calculate the insulin in body based on the insulin administration information of the first injection device 11. In this case, in one embodiment, the platform system for integrated management of insulin in body 2 may identify the type of insulin administered in the past (S203), and generate, as insulin in body information, a residual insulin in body (IOB) or an active insulin in the body value (U/min) according to the type of insulin administered in the past (S204).

Next, the platform system for integrated management of insulin in body 2 may receive injection device information corresponding to a second injection device 12 that the user will use in the future from the user device 1 or the second injection device 12 (S205). In this case, the injection device information received by the platform system for integrated management of insulin in body 2 may include the type of insulin to be administered by the second injection device 12.

The platform system for integrated management of insulin in body 2 may generate, based on the insulin in body information calculated in step S204 and the injection device information received in step S205, insulin administration information defining the amount of insulin that the second injection device 12 should administer and/or the administration time point of the insulin. Next, the platform system for integrated management of insulin in body 2 may transmit the generated insulin administration information to the second injection device 12 to automatically set the administration operation of the second injection device 12 (S206). However, this is exemplary; in other embodiments the insulin administration information may be transmitted to the second injection device 12 via the user device 1.

The continuous management operation based on the insulin in body as described above may be performed in the same manner as above even when the insulin administration means that the user uses is changed from the second injection device 12 to still another third injection device 13.

That is, the platform system for integrated management of insulin in body 2 may receive insulin administration information from the second injection device 12 (S207), and receive injection device information indicating that the third injection device 13 will be used for insulin administration in the future from the user device 1 or the third injection device 13 (S208). Next, the platform system for integrated management of insulin in body 2 may generate administration information indicating the dose and/or administration time point of insulin by the third injection device 13 based on the insulin in body resulting from the insulin administration by the second injection device 12 and the type of insulin administered by the third injection device 13, and may transmit the administration information to the third injection device 13 to set the administration information (S209).

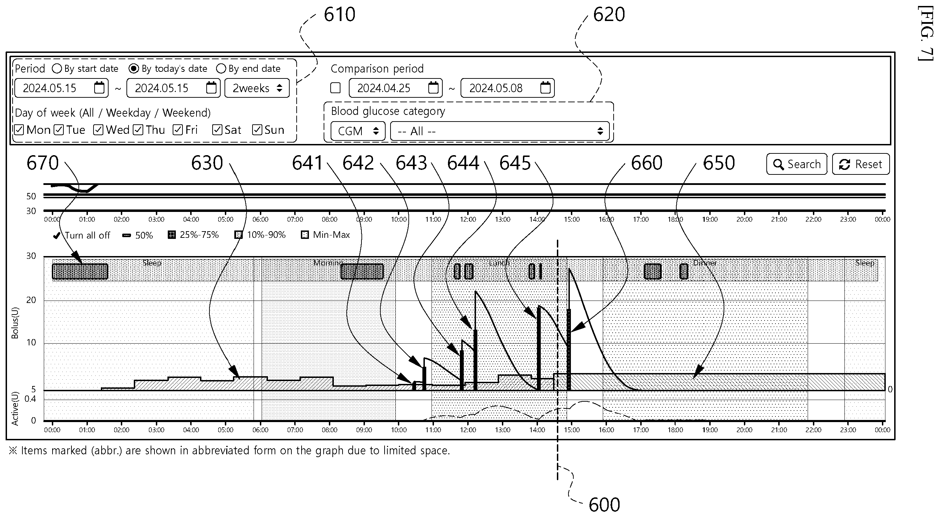

FIGS. 7 to 9 are conceptual diagrams showing an exemplary user interface (User Interface; UI) provided by a platform system for integrated management of insulin in body according to one embodiment.

Referring to FIG. 7, the platform system for integrated management of insulin in body according to one embodiment may provide a UI element 610 with which the user can designate the date and time, period, day of the week, and the like that the user wishes to query, thereby providing a function by which the user can query his or her insulin administration information by time.

In addition, the platform system for integrated management of insulin in body according to one embodiment allows the user to select a blood glucose or an insulin type through a UI element 620 or the like, and allows the administration information of insulin for managing the selected blood glucose or the administration information of the selected type of insulin to be confirmed via the screen. In this case, the insulin management may be classified into meal bolus insulin management, basal insulin management, and the like, but is not limited thereto.

The screen area displayed by the platform system for integrated management of insulin in body is allocated to the insulin administration information, and FIG. 7 exemplarily shows a case where the administration device that the user uses for insulin administration has been switched from a first administration device to a second administration device, based on a division time point 600 indicated by a dotted line. In this case, the first administration device and the second administration device mean devices having different insulin administration characteristics, such as the type of administered insulin, the insulin dose, and/or the insulin administration cycle. The device switching time point 600 may be displayed to the user as part of the UI screen so that the user can recognize the device switching.

Hereinafter, the integrated management operation of insulin in body by the platform system for integrated management of insulin in body will be described on the assumption that the first administration device is an administration device (for example, an insulin pump) that uses rapid-acting insulin for both meal bolus insulin and basal insulin, and the second administration device is an administration device (for example, an insulin pen; respective insulin pens according to the insulin type may be used) that uses rapid-acting insulin for the meal bolus and long-acting insulin for the basal insulin.

For example, there may be cases where the user must remove the first administration device from the body and switch to the insulin pen, which is the second administration device, to administer insulin, such as in the event of a malfunction of the insulin pump that is the first administration device being used, clogging of the injection mechanism, or battery depletion, or when engaging in vigorous exercise such as marathon running, cycling, or swimming, when exposure to water such as bathing or sea bathing is required, or when being on a trip or in the presence of others.

Hereinafter, the description will be divided into meal bolus insulin management and basal insulin management.

First, describing the management of meal bolus insulin, each of the bars 641 to 645 displayed on the UI screen represents the administration time point and dose of rapid-acting insulin administered in correspondence with the user's mealtimes, and the solid line that continues with decreasing values from the top of each of the bars 641 to 645 toward the right represents that, after the user's IOB increases due to the administration of rapid-acting insulin, the IOB decreases over time.

In this case, at the time point 600 at which the switching of the administration means takes place, the platform system for integrated management of insulin in body 2 calculates the user's current IOB through the dose and administration time point of insulin represented by each of the bars 641 to 645. When the current time point arrives at which rapid-acting insulin is administered in correspondence with the next mealtime after the switching of the administration means 600, the platform system for integrated management of insulin in body may calculate the final dose of insulin to be administered via the current administration means (i.e., the insulin pen) by taking into account the current IOB calculated through the prior insulin administration. This is illustrated as a bar 660 in FIG. 7, and the final insulin dose corresponding to the bar 660 may be determined based on the IOB calculation through the prior insulin administration. For example, since the user has IOB remaining in the body due to the prior insulin administration represented by the bar 645, even when the next mealtime arrives, the insulin dose may be automatically determined based on the current IOB instead of administering rapid-acting insulin at the maximum value.

In detail, as in the bar 660, when a total required insulin amount of 27 U is calculated through the meal bolus calculation formula, the current IOB at the time point of the switching of the administration means 600 is estimated as 9 U through the calculation formula with reference to the bar 645, and these are subtracted to determine 18 U as the final dose. The determined dose of 18 U may be delivered to the user as an alarm via the user device 1 or the injection devices 11 to 13, or may be displayed on a display. By administering the final dose of 18 U thus determined, a total required insulin amount of 27 U can be calculated by adding the current IOB of 9 U before administration. This IOB then decreases again toward the right from the end of the bar 660.

As an example, the amount of meal bolus insulin to be administered may be determined by taking into account the current IOB, as shown in Table 1 below.

**[Table 1]**

| **Category** | **Item** | **Value** |
|---|---|---|
| Carbohydrate-intake glucose-regulating insulin (A) | Meal category | 240 g |
| | ICR (g/U) | 10 |
| | A: Calculated amount (U) | 24 U |
| Target blood glucose-regulating insulin (B) | CF (mg/dL/U) | 35 |
| | Target blood glucose (mg/dL) | 105 |
| | Current blood glucose (mg/dL) | 210 |
| | B: Calculated amount (U) | 3 |
| Current IOB (C) | C: Calculated amount (U) | 9 |
| **Final result (A + B - C) (meal insulin to be administered)** | | **27 U** |

In Table 1 above, the carbohydrate-intake glucose-regulating insulin (A) is calculated based on the amount of meal carbohydrates directly input by the user, and the target blood glucose-regulating insulin (B) represents the amount of required insulin correspondingly calculated based on the blood glucose management target. That is, A is calculated as 24 U by dividing the meal carbohydrates of 240 g by the ICR (insulin-to-carbohydrate ratio), and B is calculated as 3 U by dividing the difference between the current blood glucose and the target blood glucose by the CF (correction factor). In this case, in the embodiment of the present invention, the meal insulin amount to be administered, 27 U, can be calculated by subtracting the current IOB (C) at the time point of the switching of the administration means 600 from the required insulin amount (A+B).

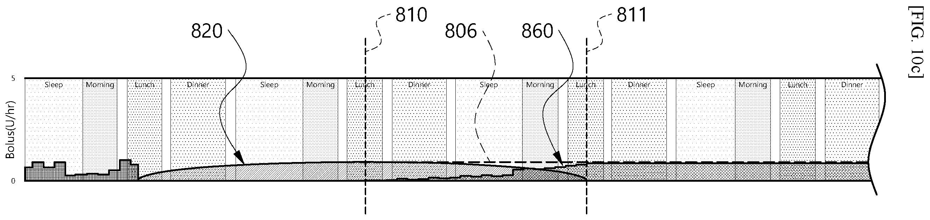

Next, describing the management of basal insulin, the graph 630 displayed on the UI screen represents the amount of basal insulin that the user administers via the insulin pump. The insulin pump is configured to continuously administer a small amount of rapid-acting basal insulin at a very short cycle (for example, 1 to 3 minutes). When a continuous glucose monitor (CGM) is used together with the insulin pump, the basal insulin dose may be determined according to the average trend of the user's blood glucose increase and decrease, and the figure shows that the basal insulin amount was increased at 3:30 a.m., 5:30 a.m., and 7:00 a.m. In addition, the y-axis bolus value of the graph 630 represents the amount of rapid-acting insulin administered per unit time. If a given time point of the graph 630 is shown at 6 U on the UI screen, the total sum of rapid-acting insulin administered during the 1 hour corresponding to that time point is 6 U, and the unit of the administration rate may be expressed as 6 U/hr. This may be understood as the insulin pump administering 0.3 U of basal insulin every 3 minutes, in which case the unit of the administration rate may likewise be expressed as 0.3 U/min. This is used identically to the unit of the target basal active insulin 806 in Embodiment 3, FIG. 10c, to be described later.

Meanwhile, in a section where the user's hypoglycemia is detected or expected via the continuous glucose monitor (CGM), the administration of basal insulin may be suspended, and the time section indicated by the rectangle 670 in FIG. 7 represents a time section in which the administration of basal insulin was suspended for this reason.

In the case of an insulin pen, the user may generally use, respectively, an insulin pen containing a rapid-acting insulin cartridge and an insulin pen containing a long-acting insulin cartridge. In meal bolus insulin management, rapid-acting insulin may be administered into the body as meal bolus insulin before a meal, and in basal insulin management, long-acting insulin may be administered into the body for a fixed period, for example once a day. In FIG. 7, the graph 650 represents the active insulin in which the long-acting basal insulin administered via the insulin pen acts constantly over its duration of action.

Meanwhile, long-acting insulin is used as basal insulin, is designed to act constantly for a certain period of time, and is intended to stably manage blood glucose by maintaining a constant basal insulin level. Accordingly, since the action of the insulin is very constant and predictable, it is mainly expressed as active insulin rather than being expressed as IOB. Such active insulin has an effect on the body in the form of a normal distribution curve, and the information of this normal distribution can be obtained by checking the manufacturer's information of the insulin product. In addition, the IOB of the relevant long-acting insulin may also be calculated through the duration of insulin activity (DIA) and the peak time (Peak Time) of the active insulin.

### [Embodiment 1] Upon switching from an insulin pump to an insulin pen

When the device that the user uses is switched from an insulin pump to an insulin pen at the time point 600 indicated by the dotted line, the platform system for integrated management of insulin in body may determine the dose and/or administration time point of meal bolus insulin and basal insulin by the insulin pen to be used in the future, by taking into account all parameters for calculating the insulin dose, such as the user's ICR, CF, and the basal insulin dose based on the past insulin administration information.

First, in terms of meal bolus insulin management, as described above, when an insulin pump using rapid-acting insulin is switched to an insulin pen using rapid-acting insulin, the platform system for integrated management of insulin in body 2 may determine the final dose of insulin to be administered via the insulin pen to which the switch is made, by taking into account the current IOB calculated through the amount of insulin previously administered via the insulin pump.

In terms of basal insulin management, in the case of the insulin pump, since basal insulin is continuously administered as a small amount of rapid-acting insulin, when the use of the insulin pump is stopped, the rapid-acting insulin is, by its nature, quickly depleted, so that there is almost no basal insulin remaining in the body; therefore, in this case, long-acting insulin may be administered as basal insulin using the insulin pen immediately after the use of the insulin pump is stopped. The graph 650 shown in FIG. 7 represents the long-acting basal insulin administered via the insulin pen as having been replaced with the active insulin that acts constantly over its duration of action.

That is, in the example shown in FIG. 7, in the case of basal insulin management, a device switch was made from an insulin pump using rapid-acting insulin to an insulin pen using long-acting insulin, and since rapid-acting insulin rapidly disappears in the body, at the switching time point 600 indicated by the dotted line, the user immediately administered long-acting insulin and switched to insulin pen management.

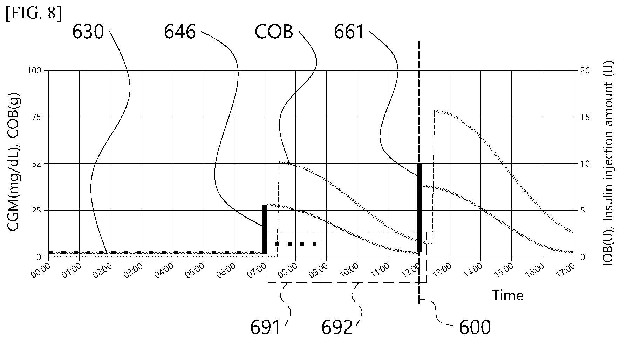

In one embodiment, the basal insulin dose by the pump may be adjusted at a time point before the switch from the insulin pump to the insulin pen is made, and FIG. 8 is an exemplary graph showing such a form of dose adjustment before the switching time point.

In FIG. 8, COB represents the amount of carbohydrates in the body that increases through a meal, the graph 630 represents the basal insulin dose by the insulin pump, and the graph 646 represents the meal bolus insulin dose by the insulin pump. Meanwhile, the graph 661 represents the meal bolus insulin dose via the insulin pen that is made immediately after the switch from the insulin pump to the insulin pen 600.

In this case, when information that the user will switch the device from the insulin pump to the insulin pen is received by the platform system for integrated management of insulin in body, the platform system for integrated management of insulin in body may, by suspending the administration of basal insulin by the insulin pump for a predetermined time section 692 before the switching time point of the administration means 600, manage the basal insulin administered by the insulin pump so that it does not remain in the body at the switching time point 600. This may also be used, for example, when there is a plan to exercise or swim in the near future, for the purpose of preventing the blood glucose level from becoming too low during exercise.

In addition, in one embodiment, the platform system for integrated management of insulin in body may increase the basal insulin dose by the insulin pump relative to normal (for example, set to twice the normal level) for a preset time section 691 before the time section 692 in which the administration of basal insulin by the insulin pump is suspended. This is to prevent the user's blood glucose from rising during the section 692 by administering more insulin in advance in preparation for a situation in which the basal insulin administration is suspended in the future.

### [Embodiment 2] Upon switching from an insulin pump to an insulin pump

In one embodiment, the injection device that the user uses may be switched from an insulin pump to another insulin pump. For example, there may be cases where the user switches to another insulin pump due to an upgrade, malfunction, or loss of the insulin pump previously being used, or where the user registers both insulin pumps in the platform system for integrated management of insulin in body and switches and inputs the pump that the user uses as needed.

In this case, first, in terms of meal bolus insulin management, the platform system for integrated management of insulin in body 2 may determine the final dose of insulin to be administered via the insulin pump to which the switch is made, by taking into account the current IOB calculated through the amount of insulin previously administered via the insulin pump.

Next, in terms of basal insulin management, since both the pre-switch administration means and the post-switch administration means are insulin pumps that continuously administer a small amount of rapid-acting insulin, basal insulin may be administered via the new insulin pump immediately after the use of the previous insulin pump is stopped, with the same target basal active insulin.

### [Embodiment 3] Upon switching from an insulin pen to an insulin pump

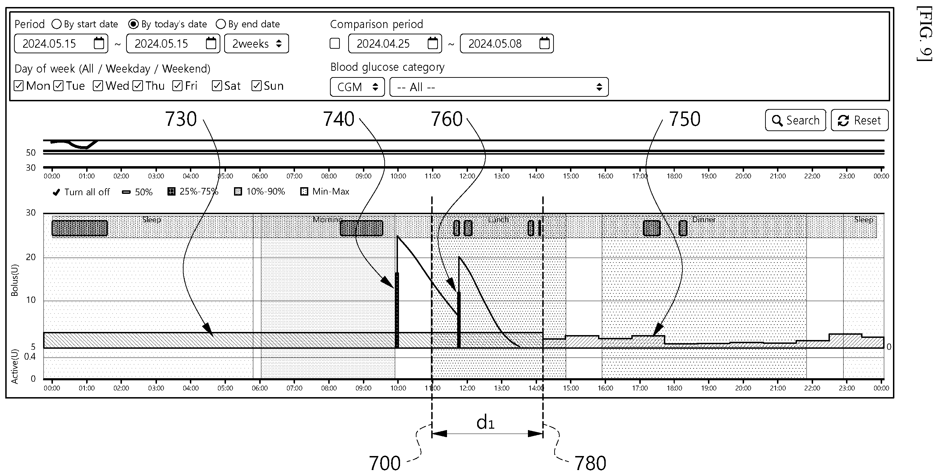

Meanwhile, a switch may be made from MDI therapy (using an insulin pen), in which meal bolus insulin is administered with (ultra-)rapid-acting insulin and basal insulin is administered with long-acting insulin, to CSII therapy (using an insulin pump), in which both meal bolus insulin and basal insulin are administered with (ultra-)rapid-acting insulin alone; in this case, the platform system for integrated management of insulin in body may select the time point at which insulin administration by the insulin pump, which is the second administration device, begins, based on the IOB calculated from the insulin already administered by the insulin pen, which is the first administration device. FIG. 9 shows an example corresponding to this.

First, describing the management of meal bolus insulin with reference to FIG. 9, the bar 740 displayed on the UI screen represents the administration time point and dose of rapid-acting insulin administered with the insulin pen in correspondence with the user's mealtimes. After the switching time point 700 of the administration device that the user uses, the platform system for integrated management of insulin in body calculates the IOB according to the rapid-acting insulin administered via the insulin pen (shown as the solid line continuing from the bar 740), and when the time point to administer the next rapid-acting insulin (for example, a mealtime) arrives, it may determine the dose of rapid-acting insulin by the insulin pump by taking into account the current IOB, which is shown as the bar 760 in FIG. 9.

Next, describing the management of basal insulin, the graph 730 displayed on the UI screen represents the amount of active insulin in which the long-acting basal insulin administered by the user via the insulin pen acts in the body, over the period during which the action effect of that insulin is constantly maintained. In this case, if the duration of action of the existing basal insulin has not ended even at the time point 700 at which the switch from the insulin pen to the insulin pump occurs, as an example the platform system for integrated management of insulin in body may integratedly manage the insulin in body in a manner that delays the administration of basal insulin by the insulin pump until the time point at which the basal insulin duration of action according to the existing insulin administration information ends.

In FIG. 9, the graph 750 represents the basal insulin dose by the insulin pump, and a predetermined time interval d1 exists between the time point 700 at which the device switch is made and the time point 780 at which the basal insulin administration by the insulin pump is made. This represents that the platform system for integrated management of insulin in body delayed the administration of basal insulin by the insulin pump until the time point at which the duration of action of the long-acting basal insulin previously administered by the insulin pen ends.

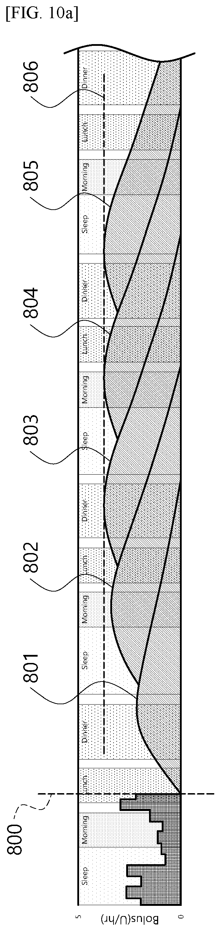

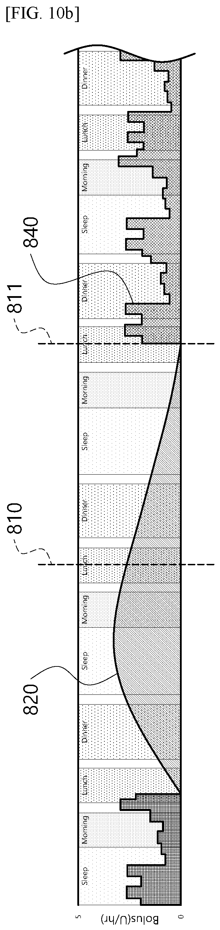

FIGS. 10a to 10d are conceptual diagrams for explaining adjustment of the basal insulin administration time point by a platform system for integrated management of insulin in body according to one embodiment; FIG. 10a shows a case where, in a state without conventional integrated management of insulin in body, the insulin pen continues to be used after switching from the insulin pump to the insulin pen, and FIGS. 10b and 10c show cases where the insulin administration time point and/or dose by the insulin pump is adjusted by integratedly managing the insulin in body according to the embodiments.

Meanwhile, the long-acting insulin used in this embodiment has a duration of action of about 3 days, has an action effect in the form of a normal distribution curve with the peak located toward the front, and decreases below a certain effect at the beginning and end of the duration of action; as an example, the description will be given of insulin degludec, a long-acting basal insulin analog, which is a basal insulin known under the trade name Tresiba and is administered once a day.

Referring to FIG. 10a, when a switch is made from the insulin pump to the insulin pen at the time point 800 indicated by the dotted line, the graphs 801 to 805 represent the periods during which the basal insulin (i.e., long-acting insulin) administered by the insulin pen acts. In this case, the active insulin stabilizes when long-acting insulin is administered on consecutive days, and in FIG. 10a, from the time the third long-acting insulin 803 is administered, the active insulin is maintained at a stable value with a constant effect. That is, FIG. 10a shows the appearance in which, when long-acting insulin having a duration of action of 3 days is administered daily, the active insulin is stabilized after about 3 to 4 days.

In this case, the user may set the stable value of the active insulin as the target basal active insulin 806. In this embodiment, the target basal active insulin 806 was set as the stable value of the active insulin according to insulin administration, and such a stable value may be a stable value at which basal insulin is administered and acts in the body according to a dose of long-acting insulin determined by a medical professional's prescription, or may be an active insulin (U/day) corresponding to administering, per day, an amount of insulin falling within a range of 40% to 60%, preferably 50%, of the patient's Total Daily Insulin Dose (TDD).

In this case, the stable value reached by administering basal insulin may mean a value at which a sum, at the present time point, of the active insulin corresponding to the basal insulin of each administration session converges, in the case where long-acting insulin is administered a plurality of times at intervals. In this specification, convergence is not limited to the sum of the active insulin necessarily becoming a particular value, and where the sum of the active insulin is maintained substantially constant within a preset margin of error, the platform system for integrated management of insulin in body may set that constant value to mean the stable value of the active insulin, that is, the target basal active insulin 806. Alternatively, the target basal active insulin 806 may be calculated, when CSII therapy (insulin pump) is used, as the average amount of basal insulin per unit time at which rapid-acting insulin is administered by the insulin pump.

In this case, as in FIG. 10b, it is assumed that the user wishes to switch from the insulin pen to the insulin pump at the time point 810 indicated by the dotted line. In FIG. 10b, the graph 820 represents, as a single line, the result of superimposing the active insulin graphs 801 to 805 shown in FIG. 10a. Conventionally, since there was no means for managing the patient's insulin in body, switching to the insulin pump at a desired time point was not recommended, and the practice of waiting until the time point 811 at which the duration of action of the previously administered basal insulin ends and then wearing the insulin pump was used as a guideline because side effects such as hypoglycemia could be minimized. That is, since it was necessary to wait until the time point 811 at which the IOB due to the insulin administered with the insulin pen is fully depleted, there has been an inconvenient problem in that the switch to the insulin pump is delayed.

On the other hand, referring to FIG. 10b, when the platform system for integrated management of insulin in body according to the embodiments is used, even if a switch is made to the insulin pump at the time point 810 before the duration of action of the basal insulin previously administered with the insulin pen ends, the administration of basal insulin by the insulin pump can be automatically delayed until the time point 811 at which the duration of action of the previously administered basal insulin ends. That is, the platform system for integrated management of insulin in body may calculate the user's IOB, automatically detect the time point 811 at which the IOB is fully depleted, and provide information to the user or automatically set the injection device so that insulin administration is performed thereafter, as shown by the graph 840.

As another example, referring to FIG. 10c, the pump may be worn immediately from the switching time point 810, while the target basal active insulin 806 according to the user's basal insulin setting is calculated and, based on this, the dose and/or administration time point of basal insulin by the insulin pump is controlled. For example, the target basal active insulin 806 may be set, as described above, as the stable value of the active insulin in the body according to the administration of long-acting insulin, but is not limited thereto.

The graph 860 of FIG. 10c represents the basal insulin administration by the insulin pump performed through such control, and the hourly administration amount of basal insulin by the insulin pump may be determined using a value obtained by calculating the current active insulin in the body according to the administration of long-acting insulin from the target basal active insulin 806 and subtracting it therefrom.

The administration of basal insulin by the insulin pump is performed in a manner of continuously administering a small amount of rapid-acting insulin as basal insulin at a very short cycle (for example, 1 to 3 minutes). In this case, the basal insulin administration amount of the insulin pump taking into account the target basal active insulin 806 is a value (in units of U/min) obtained by subtracting, from the target basal active insulin 806, the active insulin of the long-acting insulin administered with the insulin pen.

Alternatively, the basal insulin administration amount of the insulin pump may be calculated by multiplying the subtracted value by a predetermined parameter. In this case, the parameter may be a preset fixed ratio, or may include a value obtained by multiplying a fixed ratio by any one of the user's correction factor (CF), insulin-to-carbohydrate ratio (ICR), and the user's body weight, and is not limited thereto.

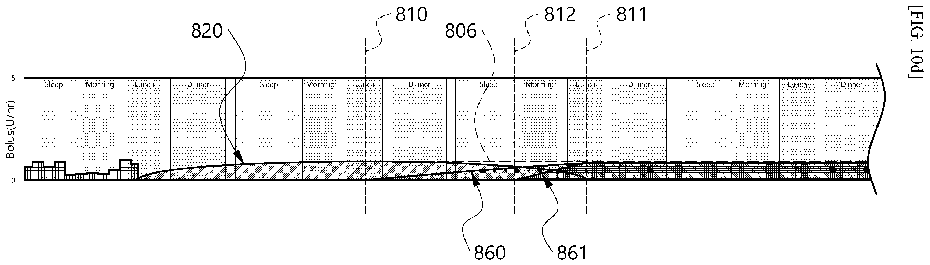

Furthermore, in still another embodiment, when the platform system for integrated management of insulin in body can know the target basal active insulin 806 but cannot obtain the current active insulin in the body value according to the administration of long-acting insulin, the hourly administration amount of basal insulin may be determined so that the hourly administration amount of basal insulin by the insulin pump linearly increases during the period between the present time point (i.e., the time point at which the administration means is switched from the insulin pen to the insulin pump) and the time point 811 at which the previously administered basal insulin is fully depleted, as shown in FIG. 10d. In this case, the time point 811 at which the basal insulin is fully depleted may be determined as the duration of action of the basal insulin.

In this case, the administration amount of basal insulin by the insulin pump may be linearly increased with, as a maximum value, the amount of basal insulin at which the active insulin in the body value corresponding to the basal insulin administered by the insulin pump reaches the target basal active insulin. However, when rapid-acting insulin that acts and is depleted in a short time after administration is used as basal insulin, the active insulin in the body corresponding to the basal insulin administered by the insulin pump may mean the amount of basal insulin administered per unit time itself.

In FIG. 10d, the graph 860 represents a case where a switch to the insulin pump is made at the time point 810 indicated by the dotted line, and the graph 861 represents a case where a switch to the insulin pump is made at another time point 812. As shown, the administration amount of basal insulin by the insulin pump may be set so that the basal insulin administration amount linearly increases from each time point of switching to the insulin pump, and reaches the target basal active insulin 806 at the time point 811 at which the previously administered basal insulin is fully depleted.

Although FIGS. 8 to 10 exemplarily describe integrated management of insulin in body in the case where a switch is made from an insulin pump to an insulin pen, or from an insulin pen to an insulin pump, it will be readily understood by a person skilled in the art that the same principle may be applied when other forms of administration means are used.

For example, there may be a case where the user uses an insulin pen for the administration of bolus insulin shown by the bars 641 to 645, 660, 740, 760 in FIGS. 7 and 9, and uses an insulin pump for the administration of basal insulin shown by the graphs 630, 650, 730, 750. Alternatively, the user, who is a diabetic patient, may simultaneously use a plurality of insulin pens or a plurality of insulin pumps in order to simultaneously use two or more types of insulin having mutually different properties (for example, bolus insulin and basal insulin). For example, the user may select the device to be used through an app that communicates with the platform system for integrated management of insulin in body, or may automatically select the device to be used through pairing of the user device and that device.

In such cases, the platform system for integrated management of insulin in body according to the embodiments may, regardless of the type of administration means that the user uses, calculate the user's insulin in body at the present time point based on the prior insulin administration information, and, based on the calculated insulin in body, adjust the dose of insulin to be administered next time (for example, in the case of bolus insulin), and/or adjust the administration time point or dose of insulin to be administered next through the target basal active insulin (for example, in the case of basal insulin).

### [Embodiment 4] Upon switching from an insulin pen to an insulin pen

In one embodiment, the injection device that the user uses may be switched from an insulin pen to another insulin pen. For example, there may be cases where the user switches to another insulin pen due to an upgrade, malfunction, or loss of the insulin pen previously being used, or where the user registers both insulin pens in the platform system for integrated management of insulin in body and switches and inputs the pen that the user uses as needed.

In this case, first, in terms of meal bolus insulin management, the platform system for integrated management of insulin in body may calculate the current insulin in body through the amount of insulin previously administered via the insulin pen, and, when meal bolus insulin administration is required via the insulin pen to which the switch is made, may determine the final administration amount of insulin by subtracting the current insulin in body from the amount of insulin to be administered.

In this case, the type of insulin and the target insulin amount that the user should administer may be automatically set by the platform system for integrated management of insulin in body or directly input by the user, and the insulin administration amount calculated by subtracting the current IOB based thereon may be determined as described above with reference to Table 1. The calculated administration amount may be provided to the user or set directly in the insulin pen. In addition, the platform system for integrated management of insulin in body may provide information about the insulin administration time point to the user or set it in the insulin pen.

In one embodiment, the platform system for integrated management of insulin in body may provide the user with information about the insulin pen that the user should use according to the determined meal bolus insulin administration amount. For example, characteristic information about the type and administration amount of insulin administered by a plurality of types of insulin pens is registered in advance in the platform system for integrated management of insulin in body, and the platform system for integrated management of insulin in body may determine the insulin pen to be used, such as whether it is possible with the currently linked insulin pen or whether the use of another insulin pen is necessary, according to the insulin administration amount calculated by reflecting the current IOB.

Next, in terms of basal insulin management, the platform system for integrated management of insulin in body may, by taking into account the duration of action and the active insulin of the long-acting insulin administered via the previous insulin pen, inform the user of the same property, administration amount, and administration time as those administered via the previous insulin pen so that the same target basal active insulin can be achieved, and thereby have the user administer long-acting insulin via the new insulin pen. The management information including the administration time point by the new insulin pen may be provided to the user or set directly in the insulin pen.

### [Embodiment 5] Case of switching from a smart pen to a general insulin injection pen

In one embodiment, there may be a case where, while using a smart pen capable of communicating with a user device 1 such as a smartphone, the user switches the injection device to a general manual insulin pen due to participation in exercise such as swimming.

In this case, in terms of meal bolus insulin management, the platform system for integrated management of insulin in body may calculate the current IOB through the administration amount and administration time point of meal bolus insulin obtained through communication between the existing smart pen and the user device, and, when administering meal bolus insulin via the manual insulin pen, may determine the final administration amount by taking into account the current IOB. Since this may be identical to the calculation process described above with reference to Table 1, a detailed description thereof is omitted. Next, in terms of basal insulin management, the platform system for integrated management of insulin in body may determine the administration amount and administration time point of long-acting insulin by the new insulin pen by taking into account the duration of action of the long-acting insulin administered by the existing smart pen. In this case, the administration amount and administration time point of basal insulin by the general insulin pen may be determined so as to achieve the same target basal active insulin as when the smart pen is used.

However, in the case of a general insulin injection pen that does not have a wireless communication function, since the platform system for integrated management of insulin in body cannot obtain information, the platform system for integrated management of insulin in body may transmit, to the user device, management information indicating the administration time point and administration amount of insulin. In addition, the platform system for integrated management of insulin in body may transmit, to the user device, notification information urging the user to input, into the user device, the administration amount and administration time point of bolus insulin and basal insulin via the insulin pen.

### [Embodiment 6] Upon switching from an insulin injection device to a medicament, or vice versa

Meanwhile, the platform system and method for integrated management of insulin in body according to the embodiments are also applicable when the means that the user uses for insulin administration is a medicament. For example, even a patient who usually regulates blood glucose with an insulin pen or an insulin pump may, when blood glucose regulation with a medicament without the use of an injection device is required in the short term, use a blood glucose-lowering agent such as a biguanide, a meglitinide, a sulfonylurea, or an SGLT-2 inhibitor. In this case, an oral blood glucose-lowering agent has a predetermined effective period for each medicament; for example, in the case of glimepiride among the sulfonylureas, it is effective for 4 to 8.7 hours, corresponding to its half-life.

Accordingly, the platform system for integrated management of insulin in body according to the embodiments may be applied to the case of switching from an oral medicament to an insulin injection device, and to the case of temporarily using an oral medicament while using an insulin injection device such as a pump or a pen. In this case, the platform system for integrated management of insulin in body may calculate the insulin in body of the user's current body based on the insulin administration information previously administered by the user, and generate management information so as to administer insulin via an insulin pen, a pump, a medicament, or the like at the time point at which the insulin in body is depleted. In this case, if the user previously administered insulin via a medicament, the operation time point of the administration means to be used for future insulin administration may be delayed based on the known effective period (for example, the half-life) of that medicament.

According to the platform system for integrated management of insulin in body and the method for integrated management of insulin in body according to the embodiments described above, users can, regardless of the type of administration means they use, monitor their blood glucose level in real time, ascertain their current state based on the insulin in body, and take appropriate measures when necessary. In addition, by the platform system for integrated management of insulin in body managing the user's insulin dose and administration time point, it can automatically set the patient's insulin administration schedule and transmit management information in the form of notifications or the like, thereby ensuring that the patient does not miss the administration schedule.

Since the above management is temporally continuous and can be continuously performed regardless of the user's insulin administration means, the insulin in body can be continuously managed even if the user temporarily uses an insulin pen for exercise such as swimming while using an insulin pump, and continuity of blood glucose management can be realized through interworking with each injection device. Since such continuous management can be performed regardless of the type of manufacturer of the injection device that the user uses, or of how many types or how many injection devices the user uses, it enables continuous blood glucose management in various environments that may arise in the daily life of a diabetic patient.

Furthermore, the platform system for integrated management of insulin in body according to the embodiments serves to help medical professionals monitor the patient's condition in real time and provide appropriate advice or medical treatment as needed, by linking the insulin administration information of a diabetic patient or the management information based on the integratedly managed insulin in body to a healthcare server such as a medical institution corresponding to that patient. In addition, the information linked between the platform system for integrated management of insulin in body and the medical institution may, as educational material related to diabetes, also help patients understand and organize their own situation.

The operations by the method for integrated management of insulin in body according to the embodiments described above may be at least partially implemented as a computer program and recorded on a computer-readable recording medium. The computer-readable recording medium on which a program for implementing the operations by the method according to the embodiments is recorded includes all kinds of recording devices in which data readable by a computer is stored. Examples of the computer-readable recording medium include a ROM, a RAM, a CD-ROM, a magnetic tape, a floppy disk, and an optical data storage device. In addition, the computer-readable recording medium may be distributed over network-connected computer systems, so that computer-readable code is stored and executed in a distributed manner. In addition, functional programs, code, and code segments for implementing the present embodiment may be readily understood by a person skilled in the art to which the present embodiment pertains.

In addition, each block or each step shown in the flowcharts of this specification may represent a module, a segment, or a part of code including one or more executable instructions for executing the specified logical function(s). Also, in some alternative embodiments, it is possible for the functions noted in the blocks or steps to occur out of order. For example, two blocks or steps shown in succession may in fact be performed substantially concurrently, or those blocks or steps may sometimes be performed in the reverse order depending on the corresponding function.

Although the present invention examined above has been described with reference to the embodiments shown in the drawings, this is merely exemplary, and a person having ordinary knowledge in the relevant field will understand that various modifications and variations of the embodiments are possible therefrom. However, such modifications should be regarded as being within the technical protection scope of the present invention. Accordingly, the true technical protection scope of the present invention should be determined by the technical spirit of the appended claims.

### [Industrial Applicability]

The embodiments relate to a platform system and method for integrated management of insulin in body, and to a computer program for the same. More specifically, the embodiments relate to a technique for providing a user with an optimal insulin management method by calculating and predicting the insulin in body based on the user's insulin administration information, and, based on the predicted insulin in body, proposing a future insulin administration plan to the user or setting it in an injection device.

## Claims

1. A platform system for integrated management of insulin in body, comprising:
a receiving module configured to receive, through a user input or through a communication connection with one or more injection devices used to administer insulin to a user, insulin administration information including at least one of a past insulin dose and administration timing of the user, and to receive administration means information to be used for future insulin administration of the user;
a management module configured to calculate the insulin in body of the user based on the insulin administration information, and, based on the insulin in body and the administration means information, to generate management information including at least one of an insulin dose and administration timing for continuous management of the insulin in body; and
an output module configured to provide the management information to the user.

2. The platform system for integrated management of insulin in body of claim 1,
wherein the receiving module is further configured to receive the insulin administration information from a first injection device communicatively connected to the platform system for integrated management of insulin in body, and
wherein the management module is further configured to set, based on the management information, at least one of an insulin dose and administration timing of a second injection device communicatively connected to the platform system for integrated management of insulin in body.

3. The platform system for integrated management of insulin in body of claim 2,
wherein the first injection device and the second injection device are devices having mutually different insulin administration characteristics,
wherein the insulin administration characteristics include at least one of a type of administered insulin, an insulin dose, and an insulin administration cycle, and
wherein the management module is further configured to:
calculate the insulin in body based on the insulin administration information by the first injection device, and
determine, based on the insulin in body and the insulin administration characteristics of the second injection device, at least one of an initial dose and an initial administration time point of insulin by the second injection device.

4. The platform system for integrated management of insulin in body of claim 1,
wherein the administration means information includes information defining the injection device or medicament to be used by the user for insulin administration, and
wherein the management module is further configured to calculate the insulin in body and the management information with respect to bolus insulin or basal insulin administered by the user.

5. The platform system for integrated management of insulin in body of claim 4,
wherein the management module is further configured to calculate the insulin in body and the management information with respect to each of bolus insulin and basal insulin administered by the user.

6. A method for integrated management of insulin in body, comprising:
receiving, by a platform system for integrated management of insulin in body, through a user input or through a communication connection with one or more injection devices used to administer insulin to a user, insulin administration information including at least one of a past insulin dose and administration timing of the user;
calculating, by the platform system for integrated management of insulin in body, the insulin in body of the user based on the insulin administration information;
receiving, by the platform system for integrated management of insulin in body, administration means information to be used for future insulin administration of the user;
generating, by the platform system for integrated management of insulin in body, based on the insulin in body and the administration means information, management information including at least one of an insulin dose and administration timing for continuous management of the insulin in body; and
providing, by the platform system for integrated management of insulin in body, the management information to the user.

7. The method for integrated management of insulin in body of claim 6,
wherein the receiving of the insulin administration information includes receiving, by the platform system for integrated management of insulin in body, the insulin administration information from a first injection device communicatively connected to the platform system for integrated management of insulin in body, and
wherein the providing of the management information includes automatically setting, by the platform system for integrated management of insulin in body, based on the management information, at least one of an insulin dose and administration timing of a second injection device communicatively connected to the platform system for integrated management of insulin in body.

8. The method for integrated management of insulin in body of claim 7,
wherein the first injection device and the second injection device are devices having mutually different insulin administration characteristics,
wherein the insulin administration characteristics include at least one of a type of administered insulin, an insulin dose, and an insulin administration cycle, and
wherein the generating of the management information includes determining, by the platform system for integrated management of insulin in body, based on the insulin in body calculated based on the insulin administration information by the first injection device and on the insulin administration characteristics of the second injection device, at least one of an initial dose and an initial administration time point of insulin by the second injection device.

9. The method for integrated management of insulin in body of claim 8,
wherein the first injection device is an insulin pump,
wherein the second injection device is an insulin pen, and
wherein the determining of at least one of the initial dose and the initial administration time point of insulin by the second injection device includes:
calculating, by the platform system for integrated management of insulin in body, based on the insulin administration information by the first injection device, a current residual insulin in body at a time point of switching to the second injection device; and
determining, by the platform system for integrated management of insulin in body, the initial dose of insulin by the second injection device by reflecting the current residual insulin in body.

10. The method for integrated management of insulin in body of claim 8,
wherein the first injection device is an insulin pump,
wherein the second injection device is an insulin pen, and
wherein the generating of the management information includes:
receiving, by the platform system for integrated management of insulin in body, information about a time point of switching from the first injection device to the second injection device; and
generating, by the platform system for integrated management of insulin in body, the management information so as to suspend insulin administration by the first injection device during a preset time interval before the switching time point.

11. The method for integrated management of insulin in body of claim 8,
wherein the first injection device is an insulin pen,
wherein the second injection device is an insulin pump, and
wherein the determining of at least one of the initial dose and the initial administration time point of insulin by the second injection device includes:
calculating, by the platform system for integrated management of insulin in body, a current residual insulin in body based on the insulin administration information by the first injection device; and
delaying, by the platform system for integrated management of insulin in body, the administration time point of insulin by the second injection device by reflecting the current residual insulin in body.

12. The method for integrated management of insulin in body of claim 8,
wherein the first injection device is an insulin pen,
wherein the second injection device is an insulin pump, and
wherein the determining of at least one of the initial dose and the initial administration time point of insulin by the second injection device includes:
calculating, by the platform system for integrated management of insulin in body, a current active insulin in the body based on the insulin administration information by the first injection device; and
determining, by the platform system for integrated management of insulin in body, the dose of insulin by the second injection device based on an amount obtained by subtracting the current active insulin in the body from a preset target basal active insulin.

13. The method for integrated management of insulin in body of claim 12, wherein the target basal active insulin is a value at which a sum of active insulin corresponding to long-acting insulin administered a plurality of times at preset time intervals by the first injection device converges.

14. The method for integrated management of insulin in body of claim 13,
wherein the determining of the dose of insulin by the second injection device includes linearly increasing, by the platform system for integrated management of insulin in body, the insulin dose by the second injection device until an active insulin in the body based on insulin administered into the body by the second injection device reaches the target basal active insulin.

15. The method for integrated management of insulin in body of claim 8,
wherein the first injection device is an insulin pen,
wherein the second injection device is an insulin pump,
wherein the insulin administration information by the first injection device includes an administration time point of long-acting insulin by the first injection device, and
wherein the determining of at least one of the initial dose and the initial administration time point of insulin by the second injection device includes determining, by the platform system for integrated management of insulin in body, the administration time point of insulin by the second injection device based on a duration of action of the long-acting insulin.

16. The method for integrated management of insulin in body of claim 8,
wherein the administration means information includes information defining the injection device or medicament to be used by the user for insulin administration, and
wherein the insulin in body and the management information are calculated with respect to bolus insulin or basal insulin administered by the user.

17. The method for integrated management of insulin in body of claim 16,
wherein the receiving of the insulin administration information includes receiving, by the platform system for integrated management of insulin in body, the insulin administration information from an injection device communicatively connected to the platform system for integrated management of insulin in body, and
wherein the providing of the management information includes:
calculating, by the platform system for integrated management of insulin in body, a current residual insulin in body of the user based on the insulin administration information; and
determining, by the platform for integrated management of insulin in body, based on the current residual insulin in body, at least one of a dose and an administration time point of a medicament that the user administers for blood glucose regulation.

18. The method for integrated management of insulin in body of claim 16,
wherein the insulin in body and the management information are calculated with respect to each of bolus insulin and basal insulin administered by the user.

19. A computer program stored in a computer-readable recording medium so as to execute, in combination with hardware, the method for integrated management of insulin in body according to any one of claims 8 to 18.

20. A method for integrated management of insulin in body that is performed by a computing device communicating with one or more injection devices so as to provide a continuous management function even when the injection device is switched, the method comprising:
receiving, through a user input or through a communication connection with a first injection device used to administer insulin to a user, first insulin administration information including a past insulin type, dose, and administration timing of the user;
calculating, based on the first insulin administration information, a residual insulin in body of the user when the past insulin is rapid-acting, and calculating an active insulin in the body of the user when the past insulin is long-acting;
receiving an insulin type corresponding to a second injection device to be used for future insulin administration of the user; and
automatically setting, in the second injection device, at least one of an insulin dose and administration timing by the second injection device, based on the calculated residual insulin in body or active insulin in the body of the user and the insulin type corresponding to the second injection device.

21. The method for integrated management of insulin in body of claim 20,
wherein the first injection device and the second injection device are devices having mutually different insulin types, the method further comprising:
receiving, through a user input or through a communication connection with the second injection device, second insulin administration information including an insulin type, dose, and administration timing corresponding to the second injection device;
calculating a residual insulin in body or active insulin in the body of the user based on the second insulin administration information;
receiving an insulin type corresponding to a third injection device, which is to be used for future insulin administration of the user and which differs in insulin type from the second injection device; and
automatically setting, in the third injection device, at least one of an insulin dose and administration timing by the third injection device, based on the residual insulin in body or active insulin in the body of the user calculated based on the second insulin administration information and the insulin type corresponding to the third injection device.

22. The method for integrated management of insulin in body of claim 21,
wherein the first injection device is an insulin pump using rapid-acting insulin,
wherein the second injection device is an insulin pen using long-acting insulin,
wherein the third injection device is an insulin pump using rapid-acting insulin,
wherein the automatically setting in the second injection device includes:
determining at least one of the insulin dose and administration timing of the second injection device, based on a residual insulin in body that is based on the rapid-acting insulin administered into the body by the first injection device and on the first insulin administration information, and
wherein the automatically setting in the third injection device includes:
determining at least one of the insulin dose and administration timing of the third injection device, based on an active insulin in the body that is based on the long-acting insulin administered into the body by the second injection device and on the second insulin administration information.

23. The method for integrated management of insulin in body of claim 20,
wherein the first injection device is an insulin pen using long-acting insulin,
wherein the second injection device is an insulin pump using rapid-acting insulin,
wherein the automatically setting in the second injection device includes determining at least one of the insulin dose and administration timing of the second injection device based on a preset target basal active insulin, and
wherein the target basal active insulin is a value at which a sum of active insulin corresponding to long-acting insulin administered a plurality of times at preset time intervals by the first injection device converges.

24. The method for integrated management of insulin in body of claim 23,
wherein the determining of at least one of the insulin dose and administration timing of the second injection device includes:
determining the insulin dose of the second injection device based on a value obtained by subtracting, from the target basal active insulin, a current active insulin in the body that is based on the long-acting insulin administered into the body by the first injection device.

25. The method for integrated management of insulin in body of claim 23,
wherein the determining of at least one of the insulin dose and administration timing of the second injection device includes:
linearly increasing the insulin dose by the second injection device until an active insulin in the body based on the rapid-acting insulin administered into the body by the second injection device reaches the target basal active insulin.
